# EUROPEAN PATENT SPECIFICATION

(11) **EP 3 573 663 B1**
(45) Date of publication and mention of the grant of the patent: **07.04.2021**
(21) Application number: 18707414.1
(22) Date of filing: 30.01.2018
(51) Int. Cl.: A61K 45/06, A61K 38/09, A61P 35/00

(54) **A COMPOSITION COMPRISING AT LEAST ONE GNRH ANTAGONIST**
ZUSAMMENSETZUNG MIT MINDESTENS EINEM GNRH-ANTAGONISTEN
COMPOSITION COMPRENANT AU MOINS UN ANTAGONISTE DE LA GNRH

(30) Priority: 30.01.2017 US 201762452096 P
(43) Date of publication of application: 04.12.2019
(60) Divisional application: 20165541.2
(73) Proprietor: Antev Limited, London W1S 1DN (GB)
(72) Inventor: LARSEN, Finn, Hawick TD9 8JS (GB)
(74) Representative: Holme Patent A/S
(86) International application number: PCT/IB2018/050559
(87) International publication number: WO 2018/138703

(56) References cited:
- WO-A1-2008/071984
- WO-A1-2013/104745
- HERBST KAREN L ED - PEA FEDERICO ET AL: "Gonadotropin-releasing hormone antagonists", CURRENT OPINION IN PHARMACOLOGY, ELSEVIER SCIENCE PUBLISHERS, NL, vol. 3, no. 6, 1 December 2003 (2003-12-01), pages 660-666, XP002561353, ISSN: 1471-4892, DOI: 10.1016/J.COPH.2003.06.009 [retrieved on 2003-10-04]
- DEBRUYNE F M J ET AL: "LHRH ANTAGONIST CETRORELIX FOR SYMPTOMATIC BPH: PROLONGED IMPROVEMENT BEYOND END OF TREATMENT IN PLACEBO-CONTROLLED TRIALS", EUROPEAN UROLOGY SUPPLEMENTS, ELSEVIER BV, NL, vol. 7, no. 3, 1 March 2008 (2008-03-01), page 171, XP022610397, ISSN: 1569-9056, DOI: 10.1016/S1569-9056(08)60397-8 [retrieved on 2008-03-01]

## Description

The present invention relates to compositions comprising at least one GNRH antagonist, and to the use of such compositions for treatment of a condition or disease related to the release of a gonadotropin hormone.

Gonadotropin-releasing hormone antagonists (GnRH antagonists) are synthetic peptides that compete with the endogenous neurohormone GnRH (otherwise known as luteinizing hormone releasing hormone, LHRH) for binding to its receptors in the anterior pituitary gland. By decreasing or blocking GnRH action, GnRH antagonists suppress release from the anterior pituitary gland of gonadotropins such as follicle stimulating hormone (FSH) and luteinizing hormone (LH).

Both FSH and LH are involved in normal reproductive function. In females, FSH stimulates the growth of immature Graafian follicles to maturation, whereas changes in LH levels control ovulation. In males, on the other hand, FSH plays an important role in spermatogenesis and LH stimulates production of testosterone in the testes.

Suppression of FSH and LH release can be used to treat a condition or disease mediated by the downstream action of the gonadotropins such as prostate cancer. This can involve surgical intervention such as surgical castration, which can be irreversible and invasive. Suppression can also be mediated through administration of a therapeutic designed to reduce the action of GnRH, and thereby suppress the downstream secretion of hormones.

One possible family of therapeutics are GnRH agonists such as leuprorelin, goserelin, triptorelin, buserelin, and histrelin. GnRH agonists have been shown to be a highly effective and safe alternative to surgical castration in the treatment of advanced prostate cancer. GnRH agonists work by overstimulating the GnRH receptor, thereby producing an initial burst of the gonadotropin. This overstimulation results in a negative feedback loop, which downregulates production of GnRH receptors, and thereby at least partially ameliorates the condition.

GnRH agonists, however, can have inherent drawbacks in their use. For instance, the administration of a GnRH agonist can produce an initial burst in the production of the gonadotropin, thereby potentially exacerbating the condition further. Furthermore, the time scale of suppression of the gonadotropin can be in the order of days, and the response to the agonist is typically not dose dependent. Administration of a GnRH agonist can also increase risk of cardiovascular disease, thereby offsetting any gains in safety produced by opting out of surgery.

GnRH antagonists are an alternative family of therapeutics to GnRH agonists, which can induce a rapid and reversible suppression of the relevant hormones without the initial stimulation (increase in secretion of gonadotropins and sex steroids) characteristics of GnRH agonists.

A GnRH antagonist can compete with natural gonadotropin releasing hormones for binding to pituitary receptors, which can suppress gonadotropins. Such suppression in men can reduce hormones in men such as testosterone and dihydrotestosterone. In some instances, such suppression obtained with a GnRH antagonist can be immediate. In exemplary short-term studies, GnRH antagonists appear to suppress LH to a considerably greater degree than FSH. However, exemplary long-term studies indicate suppression of both LH and FSH. In some instances, the duration of hormone suppression after administration of a GnRH antagonist can be dose-dependent in contrast to the use of a GnRH agonist. Furthermore, the use of a GnRH antagonist can produce a more rapid recovery after cessation of use than a GnRH agonist, and carries a lower overall risk of cardiovascular disease than a GnRH agonist.

In order to effectively reduce the level of gonadotropins, it is preferred that the concentration of a GnRH antagonist in the subjects plasma remains substantially constant and above a threshold level. In this respect sustained release compositions are beneficial, since it is possible to ensure a more uniform and sufficient plasma drug profile, and a smoother therapeutic response over the dosage interval (provided the time-course of drug effects reflects the plasma concentration-time profile).

Clinically, this offers the potential to optimise drug therapy and decrease the occurrence of concentration-related adverse effects, reduce the exposure to drug and reduce the cost of drug therapy. In addition, sustained release formulations may increase patient acceptance and compliance of therapy. A sustained release formulation of a shorter-acting drug that provides a 'residual' therapeutic response at the end of the dosage interval can provide additional 'cover' in comparison with a conventional (immediate release) formulation.

A number of GnRH antagonists are known in the art, however a problem with these known GnRH antagonists is that they are not freely soluble in water or in other solvents and they have a propensity to form gels even at low concentrations (Ref: J. Med. Chem., 2001, 44, 453-467). This is due to the fact that GnRH antagonists are hydrophobic peptides, and consequently has a tendency to form gels in the presence of counter-ions.

Sustained release formulations usually require very high amounts of the GnRH antagonist dissolved in small volumes water or some other suitable solvent(s), and the relatively low solubility of the GnRH antagonist and their concentration-dependent propensity to form gels in aqueous or other solvents greatly limit their use in sustained release formulations.

WO 2003/022243 discloses microcrystalline suspensions of the GnRH antagonist teverelix [Ac-D-Nal-D-pClPhe-D-Pal-Ser-Tyr-D-Hci-Leu-Lys(iPr)-Pro-D-Ala-NH₂] in water, which suspension provide for release of teverelix over a prolonged period. However, a single injection of said suspension will not provide a sufficient immediate release of GnRH antagonist, which is desirable in order to obtain a fast reduction of the gonadotropins, e.g. if medical castration of the subject is desired.

Thus, there is a demand for a composition and an administration method of said composition that reduces the variability of the release rate of the drug over time and increases the duration on effect over time; and is efficacious in the treatment of condition or disease mediated by the downstream action of the gonadotropins.

Thus, it is a first aspect of the present invention to provide a composition containing at least one GnRH antagonist, which is arranged for prolonging the circulatory half-life of the GnRH antagonist, thereby prolonging the duration of a therapeutic effect of the GnRH antagonist *in vivo.*

It is a second aspect of the present invention to provide a composition having improved pharmacokinetics.

It is a third aspect of the present invention to provide a composition suitable for establishing therapeutically effective plasma levels of a GnRH antagonist for a period of at least about 28 days.

It is a fourth aspect of the present invention to provide a composition having a longer release rate and at the same time a fast onset of action.

It is a fifth aspect according to the present invention to provide a composition that is cheaper to manufacture and relatively simple and reliable to administer.

These and further aspects are achieved according to the present invention by providing a composition comprising at least one GnRH antagonist or a salt thereof for the treatment of a condition or disease related to the release of a gonadotropin hormone, wherein the administration of said composition comprises independently administering a therapeutically effective amount of a first GnRH antagonist or a salt thereof via a first route of administration, and a therapeutically effective amount of a second GnRH antagonist or a salt thereof via a second route of administration, and wherein the first and the second route of administration is different, wherein the first and second GnRH antagonist or a salt thereof is administered substantially simultaneously or within a period of not more than 24 hours, and wherein the first route of administration is a subcutaneous administration.

The first route of administration is preferably a subcutaneously administration and the second route of administration is preferably an intramuscular administration.

The dual administration, i.e. the administering of a therapeutically effective amount of the GnRH antagonist or a salt thereof via two different routes of administration, i.e. intramuscularly and subcutaneously, has been proven to improve the PK/PD profile of the respective GnRH antagonist(s). As an example can be mentioned that the dual administration will produce a surprising and unexpected prolonging of the circulatory half-life of the respective GnRH antagonist(s) relative to the individual injections alone, thereby prolonging the duration of a therapeutic effect of the GnRH antagonist(s) *in vivo.*

The inventors have further shown that the dual administration prevents the formation of a "dead"-phase in which insufficient GnRH antagonist is released. Consequently the composition provides a continuous, more uniform and appropriate release of the GnRH antagonist than hitherto known.

The different administrations routes can be performed in any suitable order. For example, the intramuscularly administration be performed before the subcutaneously administering, or the administration order may be reversed, i.e. the GnRH antagonist is administered subcutaneously before the intramuscularly administration. It is however preferred that the first and second GnRH antagonist is administered via the first and second route of administration substantially simultaneously, i.e. as simultaneously as is practically possible for the person performing the administration. However, in order to obtain the prolonged circulatory half-life of the respective GnRH antagonist(s), the separation period between the two administrations is less than 24 hours.

In one embodiment, the first and second GnRH antagonist or a salt thereof is administered within a period of not more than 6 hours, preferably within a period of not more than 1 hour and even more preferred within a period of not more than 1 to 5 minutes.

The intramuscularly and subcutaneously administering may in a preferred embodiment be effected by injection, as this ensures a simple and effective administration route.

Furthermore, the composition according to the invention is not only capable of providing a sustained and controlled release of the antagonist over time, the combination of the two different administration routes has surprisingly ensured that even lower concentrations of total GnRH antagonist is required in order to ensure that the concentrations of the gonadotropins in the subjects plasma is maintained at therapeutic levels/concentrations.

The first and second GnRH antagonist or salt thereof may in a simple embodiment be the same, however the respective GnRH antagonist or salt thereof may equally well be different. The choice of GnRH antagonist(s) may e.g. depend on the condition or disease to be treated as well as the desired release profile of the GnRH antagonist. However, irrespectively of the used GnRH antagonists, the composition according to the invention has proven to provide a more reliable sustained delivery of the antagonist in a more appropriate release profile.

The inventors of the present invention has found that a therapeutically effective amount of the first and second GnRH antagonist can independently comprise a dose ranging from about 30 mg to about 240 mg of the GnRH antagonist or salt thereof.

If the first administration is intramuscularly and the second administering is subcutaneously (or vice versa), said two administrations may each independently comprise a dosage of about 90 mg of the respective GnRH antagonist(s) or salt thereof, i.e. the GnRH antagonist can be administered at a total dosage of about 180 mg.

The inventors of the present invention has found that by performing a dual intramuscularly and subcutaneously administration of a therapeutically effective amount of the respective GnRH antagonist(s) or a salt thereof substantially simultaneously, said administration can produce a maximal plasma concentration (Cmax) of at least about 10 ng/mL and an area under the plasma concentration versus time curve from time 0 to time t (AUC₍₀₋ₜ₎) from about 100 ng*h/mL to about 8,000 ng*h/mL when administered at a total dose of from about 60 mg to about 480 mg, and where t can be at least about 28 days.

In a further preferred embodiment the dual administering of the respective GnRH antagonist(s) or salt thereof can produce a Cmax of at least about 15 ng/mL and an AUC(₀₋ₜ) from about 1,000 ng*h/mL to about 3,000 ng*h/mL when administered at a total dose of from about 120 mg to about 240 mg, and where t can be at least about 28 days, and in an even more preferred embodiment the dual administering of the respective GnRH antagonist (s) or salt thereof can produce a Cmax of at least about 19 ng/mL and an AUC(0-t) from about 1,000 ng*h/mL to about 3,000 ng*h/mL when administered at a total dose of about 240 mg and a dosing interval of about 28 days.

Thus, the dual administration provides a fast release of the GnRH antagonist ensuring that the gonadotropin-releasing hormone (GnRH) receptors in the pituitary gland are blocked, such that a rapid and sustained suppression of the hormones are achieved.

The dual administration further ensures that the blood plasma concentration of the respective GnRH antagonist(s) or salt thereof does not fall below about 0.5 ng/mL, preferable not below about 5 ng/mL for a period of time from about 6, 12, 18, 24, 30, 36, 42, 48, 54, 60, 66, 72, 78, 84, 90, 96, 102, 108, 114, or 120 hours after the administration. Preferably, the blood plasma concentration of the respective GnRH antagonist(s) or salt thereof lies in an area between about 0.5 ng/mL to about 10 ng/mL, and is preferably at least about 5 ng/mL in order to ensure a therapeutically effective concentration of the GnRH antagonist(s) in order to maintain an effective treatment during a treatment period.

In a preferred embodiment an additional dosage of a therapeutically effective amount of a GnRH antagonist or salt thereof is administered subsequent to the dual administration, e.g. as part of a treatment course comprising a defined dosing frequency or a dosing interval.

Accordingly, the additional dosage of the GnRH antagonist can in some instances be administered at regular time intervals for an indefinite amount of time for the treatment of a chronic condition or disease, or the additional dosage of the GnRH antagonist can be administered intermittently as needed.

However, in a preferred embodiment the dosage interval for administering the additional dosage can be at least about 28 (4 weeks) days after the preferably simultaneously dual administering i.e. the intramuscularly and subcutaneously administering. Alternatively, the additional dosage can be administered every six weeks (42 days) or every eight weeks (56 days) .

The additional dosage may comprise the same GnRH antagonist as either the first and/or second GnRH antagonist or salt thereof, but said additional dosage may also comprise an GnRH antagonist which is different from said first and second GnRH antagonists.

The additional dosage preferably comprises about 90 mg of the GnRH antagonist or salt thereof, as said dosage has proven effective in maintaining the GnRH antagonist plasma concentration at a desired (e.g. above 5 ng/mL) and therapeutically concentration for a period of at least 28 days.

The GnRH antagonist or salt thereof is in an advantageously embodiment a peptide and may e.g. be in the form of a solution, an emulsion, a gel, or a suspension. In some exemplary embodiments, a GnRH antagonist can comprise a crystalline suspension, preferably a microcrystalline peptide suspension. WO 2003/022243 discloses microcrystalline suspensions of the GnRH antagonist teverelix [Ac-D-Nal-D-pClPhe-D-Pal-Ser-Tyr-D-Hci-Leu-Lys(iPr)-Pro-D-Ala-NH₂] in water, which suspension provide for release of teverelix over a prolonged period.

If the first and a second GnRH antagonist or salt thereof is different, the first and the second GnRH antagonist or salt thereof can be independently in a crystalline form, where the crystalline form can be in a suspension or in a solution. In some instances, the first GnRH antagonist or salt thereof and the second GnRH antagonist or salt thereof independently comprise a microcrystalline peptide in suspension.

In some instances, the salt of the GnRH antagonist can comprise a counter-ion, where the counter-ion can be the conjugate base of an acid. It is preferred that the acid has a pKa value of less than about 2, however other suitable pKa values is also contemplated within the scope of protection. Accordingly, the acid can have a pKa of between 1.0 and 7.0. In a preferred embodiment the counter-ion are the conjugate base of sulfuric acid, trifluoromethanesulfonic acid, trifluoroacetic acid, or benzenesulfonic acid.

The respective GnRH antagonist(s) or salt may individually be abarelix, cetrorelix, degarelix, ganirelix, ozarelix, antide, teverelix, or a salt of any of these. It is however preferred that the GnRH antagonist is teverelix [Ac-D-Nal-D-pClPhe-D-Pal-Ser-Tyr-D-Hci-Leu-Lys(iPr)-Pro-D-Ala-NH₂] or a salt thereof. Teverelix is a synthetic decapeptide with a water solubility of approximately 10 mg/mL.

Formulations comprising many counter ions of teverelix can form gels. However, it is advantageous to formulate and administer a solution or microcrystalline suspension of teverelix, as such formulations can provide both immediate and prolonged release depending on the specific formulation. WO 2003/022243 discloses production of strong acid salts of teverelix, such as triflouroacetic acid and sulfuric acid. Such salts can provide release of teverelix over a prolonged period of time. Alternatively, administration of the acetate salt of teverelix can provide immediate release when administered subcutaneously. In some exemplary embodiments, the GnRH salt administered is teverelix trifluoromethanesulfonate, teverelix triflouroacetate, teverelix benesulfonate, teverelix acetate, or a combination thereof.

In a preferred embodiment, the GnHR antagonist is teverelix trifluroacetate (TFA), since Teverelix TFA has inherent advantages over other GnRH antagonists, including a lower risk of local irritation or anaphylactic reactions. For example, teverelix TFA has been shown to produce less local irritation at the administration site than degarelix, as well as display of a more dose-dependent response to administration.

In a preferred embodiment, the first GnRH antagonist or salt thereof and the second GnRH antagonist or salt thereof independently can comprise GnRH antagonist crystals suspended in solution. In some instances, the first GnRH antagonist or salt thereof and the second GnRH antagonist or salt thereof can independently comprise a microcrystalline aqueous suspension comprising teverelix trifluoroacetate. In some instances, the microcrystalline aqueous suspension comprising teverelix trifluoroacetate when lyophilized can be stable for at least a year when stored in a sealed container at a temperature of from about 2 °C to about 8 °C at 50% relative humidity as measured by the amount of teverelix after storage compared to prior to storage, where the teverelix amount e.g. can be determined by HPLC e.g. with a UV-detector.

The respective GnRH antagonists described herein can also be incorporated into microparticles as e.g. disclosed in WO2001/54662. For instance, the microparticle may be a microsphere such as a hollow microsphere used as a carrier for the GnRH antagonists or salt thereof. The GnRH antagonist may alternatively or in addition be formulated within a nanoparticle. The microparticle or nanoparticle may be made of a plastic, a metal, or a polymer.

As discussed earlier the dual administration will produce a surprising and unexpected prolonging of the circulatory half-life of the respective GnRH antagonist(s) relative to the individual injections alone, thereby prolonging the duration of a therapeutic effect of the GnRH antagonist(s) *in vivo.* The inventors of the present invention has in this respect shown that if the first and second GnRH antagonist is teverelix trifluoroacetate a dual administration according to the present invention will provide a blood plasma teverelix concentration in the subject of at least about 9 ng/mL and/or a blood plasma Cmax of teverelix in the subject of at least about 19 ng/mL and/or a AUC₍₀₋ₜ₎ of teverelix in the subject of at least about 1,000 ng*h/mL, where t can be at least about 28 days after the dual administration, i.e. the first administering and the second administering.

Said dual administration will further ensure that the blood plasma teverelix concentration in the subject remains above at least about 5 ng/mL from about: 24, 48, or 72 hours after the dual administering until a subsequent additional dosage of a therapeutically effective amount of an GnRH antagonist, preferably teverelix trifluoroacetate, is administered.

Thus, the composition according to the invention has both an immediate onset of action leading to a fast suppression of the gonadotropins, but also a sustain release of antagonist thereby ensuring that the subject maintains a therapeutically effective concentration in the blood plasma. This will not only provide a more reliable composition for the treatment of gonadotropin relates diseases and conditions, but also improve patient compliances as fewer administrations e.g. injections are required.

A composition according to the invention comprising teverelix TFA as the first and second GnRH antagonist, is especially suitable for treating a condition such as prostate cancer through a suppression of gonadotropins such as testosterone and dihydrotestosterone (DHT). Since such a composition both will have an immediate onset of action leading to a profound suppression of testosterone, and a sustain release of antagonist thereby ensuring that the subject maintains chemically castrated, the composition is both valuable in the treatment of patients with prostate cancer where fast control of disease is needed and for patients where only sustained release is relevant.

The composition according to the invention may however equally well be used to at least partially ameliorating other diseases or condition related to the release of a gonadotropin hormone. Said disease or condition may be benign prostatic hyperplasia; acute urinary retention; endometriosis; a cancer such as prostate, breast, or cervical cancer; a hormone imbalance; an androgen-sensitive condition; an estrogen sensitive condition; or a combination thereof.

If the disease or condition is androgen-sensitive condition the disease or condition can be benign prostatic hyperplasia, acute urinary retention, a cancer, or any combination thereof. In some instances, the disease or condition may not be benign prostatic hyperplasia, acute urinary retention, a cancer e.g. prostate cancer, or any combination thereof. In some instances, the dual administration may lead to a chemical castration.

As an example of the benefits of the dual administration of the composition according to the invention the prostate size may be reduced in the subject after said dual administration. Similarly, the urine flow may be increased in the subject after the dual administering, e.g. intramuscularly and subcutaneously.

Even though the subject may be any kind of primate, it is preferred that the subject is human.

When the disease or condition is androgen-sensitive condition the dual administration of the composition according to the invention may effectively reducing blood plasma testosterone level in a subject, where the blood plasma testosterone level can be reduced relative to a blood plasma testosterone level before the dual administration. In some instances, the testosterone can be elevated as a result of hyperthyroidism, Grave's disease, precocious puberty, or cancer. Similar the composition can be used as a means for birth control.

In this respect the inventors have shown that the dual administration, e.g. the administering intramuscularly and the administering subcutaneously may provide a blood plasma testosterone concentration less than about: 3 ng/mL, 2.5 ng/mL, 2 ng/mL, 1.5 ng/mL, 1 ng/mL, or 0.5 ng/mL as measured by mass spectroscopy. In some instances, the dual administering e.g. intramuscularly and subcutaneously, provide a blood plasma testosterone concentration less than about 0.5 ng/mL. In some instances, a blood plasma testosterone concentration of less than about 0.5 ng/mL can be achieved and maintained from about 24, 48, or 72 hours after the dual administering.

It is however preferred that the low blood plasma testosterone concentration is maintained below about 0.5 ng/mL during the entire dosing interval of at least about 28 days, even more preferred at least 42 days and even more preferred at least 56 days, i.e. until an additional dosage of the GnRH antagonist is administered, preferably subcutaneously.

The dual administrating will in addition to reducing the blood plasma testosterone concentration preferably also reduce the subject's blood plasma prostate specific antigen (PSA) concentration to less than about 4 ng/mL. PSA is a substance made by cells in the prostate gland (both normal cells and cancer cells). PSA is mostly found in semen, but a small amount is also found in the blood. Incidence of an enlargement of a prostate, such as through a condition such as BPH or prostate cancer, can increase an amount of PSA in a subject. Therefore, PSA levels can be used as a metric for treatment of a condition resulting in the increase in PSA levels.

Accordingly, the composition according to the present invention may be used for chemical castration, and in which the dual administration of the composition comprises:
(a) administering intramuscularly of teverelix trifluoroacetate to a subject; and
(b) administering subcutaneously of teverelix trifluoroacetate to the subject,
wherein the administering intramuscularly and the administering subcutaneously can be performed substantially simultaneously or at least within about 24 hours, and wherein a blood plasma testosterone concentration in the subject of less than about 0.5 ng/mL can be achieved by about 48 hours after the administering intramuscularly and the administering subcutaneously.

If the intramuscularly and subcutaneously administration each independently comprise a dosage of 90 mg teverelix TFA, a plasma teverelix concentration of at least 9 ng/mL is obtained for a period of at least about 72 hours whereby a fast medical castration of the subject is provided. Thereafter the composition will have a sustained release of teverelix TFA maintaining the plasma teverelix concentration above about 5 ng/mL ensuring that the subject will remain castrated until the next administration of teverelix TFA, e.g. 28 days later.

If disease or condition is an estrogen-sensitive condition, the composition is preferably used for treating breast cancer and for reducing pain associated with endometriosis. In this respect, the dual administration will preferably reduce the blood plasma estrogen level in a subject, where the blood plasma estrogen level can be reduced relative to a blood plasma estrogen level before administration of the composition. It is preferred that the respective estrogen is an estradiol.

The invention also relates to embodiments in which an additional pharmaceutical substance is administered. Said additional pharmaceutical substance can e.g. be co-administered with the dual administration of the respective GnRH antagonist, with the subsequent additional administration of a GnRH antagonist, or independently of the administration of the GnRH antagonist, e.g. prior to the dual administration or after.

The additional pharmaceutical substance may be any substance capable of providing a beneficial effect on the condition or disease to be treated by the composition according to the invention. As an example can be mentioned that the additional pharmaceutical substance may be an antineoplasticor, an adrenergic receptor antagonist e.g. an alpha-blocker or a beta-blocker, and/or a 5α-reductase inhibitor.

The present invention also relates to a pharmaceutical composition comprising the composition according to the invention, and in which the pharmaceutical composition further comprises a pharmaceutically acceptable excipient, diluent, or carrier. For instance, at least one of the first GnRH antagonist or salt thereof; or the second GnRH antagonist or salt thereof can be in a formulation further comprising a pharmaceutically acceptable excipient diluent, or carrier.

The diluent may in a simple embodiment be water but can also be an aqueous buffering agent, e.g. saline, citrate, phosphate, acetate, glycine, tris(hydroxymethyl)aminomethane (tris), 4-(2-hydroxyethyl)-1-piperazineethanesulfonic acid (HEPES) and/or piperazine-N,N'-bis(2-ethanesulfonic acid) (PIPES).

In some embodiments, the first GnRH antagonist or salt thereof and the second GnRH antagonist or salt thereof can be in a formulation further comprising an isotonic agent, e.g. a polyhedric alcohol, a sugar alcohol and/or mannitol.

The present invention also relates to a kit comprising at least one GnRH antagonist. The kit may further comprise means to perform a dual injection as described herein, as well as instructions for performing the dual injection.

In some aspects, the GnRH antagonist or salt thereof can be packaged in a container and the kit can further comprise at least one syringe and needle for administration of a GnRH antagonist. In some exemplary embodiments, a pair of needles can be provided of proper length for injection via intramuscular and subcutaneous injection. In some instances, a single needle can be used to administer a GnRH antagonist subcutaneously and intramuscularly. Instructions for such an administration can be packaged in the kit.

In some instances, a GnRH antagonist can be provided as a lyophilized sample contained in a vial. A kit can comprise a vial of sterile water for reconstitution/resuspension of the lyophilized GnRH antagonist, as well as instructions for preparation. In other instances, a syringe comprising an integrated water chamber can be provided to re-suspend the lyophilized GnRH antagonist within the syringe prior to administration.

Methods of making the kit can include placing at least one GnRH antagonist described herein or a salt thereof in a container for packaging. The method of making the kit can further comprise the inclusion of instructions for use. In some cases, the instructions for use can provide instructions for treating a disease or condition, or a symptom thereof.

### Definitions:

The terminology used herein is for the purpose of describing particular cases only and is not intended to be limiting.

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" can mean within an acceptable error range for the particular value as determined by one of ordinary skill in the art, which will depend in part on how the value is measured or determined, i.e., the limitations of the measurement system. For example, "about" can mean about plus or minus 10%, per the practice in the art. Alternatively, "about" can mean a range of up to 20%, up to 10%, up to 5%, or up to 1% of a given value. Alternatively, particularly with respect to biological systems or processes, the term can mean within an order of magnitude, within 5-fold, or within 2-fold, of a value. Where particular values are described in the application and claims, unless otherwise stated the term "about" meaning within an acceptable error range for the particular value should be assumed. Also, where ranges and/or subranges of values are provided, the ranges and/or subranges can include the endpoints of the ranges and/or subranges.

Within the context of the present invention recitation of the term/phrase "GnRH antagonist" are to construed as including a salt thereof, even if not explicitly recited.

The term "subject", "patient" or "individual" as used herein in reference to an individual, and can encompass a mammal and a non-mammal. A mammal can be any member of the Mammalian class, including but not limited to a human; a non-human primates such as a chimpanzee, an ape or other monkey species; a farm animal such as cattle, a horse, a sheep, a goat, a swine; a domestic animal such as a rabbit, a dog (or a canine), and a cat (or a feline); or a laboratory animal including a rodent, such as a rat, a mouse and a guinea pig, and the like. A non-mammal can include a bird, a fish and the like. In some embodiments, a subject can be a mammal. In some embodiments, a subject can be a human. In some instances, the human can be an adult. In some instances, the human can be a child. In some instances, the human can be age 0-17 years old. In some instances, the human can be age 18-130 years old. In some instances, the subject can be a male. In some cases, a male can comprise a subject born with at least a portion of a prostate. In some cases, a male can comprise a subject born with a Y chromosome. In some instances, a male can comprise a subject born with male gonads. In some instances, the subject can be a female. In some cases, a female can comprise a subject born without at least a portion of a prostate. In some cases, a female can comprise a subject born without a Y chromosome. In some instances, a female can comprise a subject born with female gonads. In some instances, a female can comprise a subject born with an ovary. In some instances, the subject is diagnosed with, or is suspected of having, a condition or disease such as cancer. In some instances, the subject can be a sexual offender. In some instances, the subject can be a pedophile. In some instances, the subject can have at least a portion of a prostate. In some cases, the subject can have at least a portion of an endometrium.

The terms "treat, " "treating", "treatment," "ameliorate" or "ameliorating" and other grammatical equivalents as used herein, can include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition, and are intended to include prophylaxis. The terms can further include achieving a therapeutic benefit and/or a prophylactic benefit. Therapeutic benefit can mean eradication or amelioration of the underlying disease being treated. Also, a therapeutic benefit can be achieved with the eradication or amelioration of one or more of the physiological symptoms associated with the underlying disease such that an improvement can be observed in the patient, notwithstanding that, in some embodiments, the patient can still be afflicted with the underlying disease.

The terms "effective amount", "therapeutically effective amount" or "pharmaceutically effective amount" as used herein, can refer to a sufficient amount of a compound being administered which will at least partially ameliorate a symptom of a disease or condition being treated.

The terms "co-administration", "administered in combination with" and their grammatical equivalents or the like, as used herein, can encompass administration of selected therapeutic agents to a single subject, and can include treatment regimens in which the agents are administered by the same or different route of administration or at the same or different times. In some embodiments, a compound disclosed herein can be co-administered with other agents. These terms can encompass administration of two or more agents to a subject so that both agents and/or their metabolites are present in the subject at the same time. They can include simultaneous administration, administration at different times, and/or administration in a composition in which both agents are present. Thus, in some embodiments, a compound and another agent(s) can be administered in a single composition. In some embodiments, a compound and another agent (s) can be admixed in the composition. In some embodiments, the same compound can be administered via a combination of different routes of administration.

As used herein, the term "bioavailability" can denote the degree to which a drug or other substance becomes available to the target tissue after administration.

Parameters often used in pharmacokinetic (PK) studies can include Tmax, Cmax, AUC(0-∞), AUC(0-t), and T_{1/2} and CL/F. "Tmax" can refer to the time to reach the maximal plasma concentration ("Cmax") after administration of a therapeutic; "AUC(0-∞)" can refer to the area under the plasma concentration versus time curve from time 0 to infinity; "AUC(0-t)" can refer to the area under the plasma concentration versus time curve from time 0 to time t; "T_{1/2}" can refer to a half-life of a therapeutic in blood plasma; "T_{1/2}, ₑₗᵢₘ" can refer to the half-life of elimination of the therapeutic from circulation; and "CL/F" can refer to an apparent clearance rate of a therapeutic.

### Gonadotropin Releasing Hormone Antagonist Formulations

In some cases, the GnRH antagonist or salt thereof can be abarelix, cetrorelix, degarelix, ganirelix, ozarelix, antide, teverelix, or a salt of any of these. A GnRH antagonist can be a salt thereof. In some instances, recitation of the phrase "GnRH antagonist" should be construed to include a salt thereof even if not explicitly recited.

In some instances, a salt can include a carboxylate salt (e.g. formate, acetate, trifluoroacetate, trichloroacetate, propionate, isobutyrate, heptanoate, decanoate, caprate, caprylate, stearate, acrylate, caproate, propiolate, ascorbate, citrate, glucuronate, glutamate, glycolate, α-hydroxybutyrate, lactate, tartrate, phenylacetate, mandelate, phenylpropionate, phenylbutyrate, benzoate, chlorobenzoate, methylbenzoate, hydroxybenzoate, methoxybenzoate, dinitrobenzoate, o-acetoxybenzoate, salicylate, pamoate, nicotinate, isonicotinate, cinnamate, oxalate, malonate, succinate, suberate, sebacate, fumarate, malate, maleate, hydroxymaleate, hippurate, phthalate or a terephthalate salt); a halide salt (e.g. chloride, bromide or iodide salts); a sulfonate salt (e.g. benzene sulfonate, methyl-, bromo- or chlorobenzenesulfonate, xylenesulfonate, methanesulfonate, trifluoromethanesulfonate, ethanesulfonate, propanesulfonate, hydroxyethanesulfonate, 1- or 2- naphthalene-sulfonate or 1,5-naphthalenedisulfonate salts); a sulfate salt; a pyrosulfate salt; a bisulfate salt; a sulfite salt; a bisulfite salt; a phosphate salt; a monohydrogenphosphate salt; a dihydrogenphosphate salt; a metaphosphate salt; a pyrophosphate salt; a nitrate salt; and the like.

In some instances, a salt can be a pharmaceutically acceptable salt. In some instances, the pharmaceutically acceptable salt can be a salt described in Berge et al, J. Pharm. Sci, 1977. In some instances, a pharmaceutically acceptable salts can include those salts prepared by reaction of a GnRH antagonist with a mineral, organic acid or inorganic base, such salts including, acetate, acrylate, adipate, alginate, aspartate, benzoate, benzenesulfonate, bisulfate, bisulfite, bitartrate, bromide, butyrate, butyn-1,4-dioate, camphorate, camphorsulfonate, caproate, caprylate, chlorobenzoate, chloride, citrate, cyclopentanepropionate, decanoate, digluconate, dihydrogenphosphate, dinitrobenzoate, dodecylsulfate, ethanesulfonate, formate, fumarate, glucoheptanoate, glycerophosphate, glycolate, hemisulfate, heptanoate, hexanoate, hexyne-1,6-dioate, hydroxybenzoate, •-hydroxybutyrate, hydrochloride, hydrobromide, hydroiodide, 2-hydroxyethanesulfonate, iodide, isobutyrate, lactate, maleate, malonate, methanesulfonate, mandelate. metaphosphate, methanesulfonate, methoxybenzoate, methylbenzoate, monohydrogenphosphate, 1-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, succinate, suberate, sebacate, sulfonate, tartrate, thiocyanate, tosylate, undeconate and xylenesulfonate.

In some embodiments, the GnRH antagonist can comprise a counter ion that is a conjugate base of an acid. In some instances, the acid can have a pKa of about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or 7.0. In some instances, the acid can have a pKa of less than about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or 7.0. In some instances, the acid can have a pKa of from about 1.0 to about 7.0, from about 1.5 to about 7.0, from about 2.0 to about 7.0, from about 2.5 to about 7.0, from about 3.0 to about 7.0, from about 3.5 to about 7.0, from about 4.0 to about 7.0, from about 4.5 to about 7.0, from about 5.0 to about 7.0, from about 5.5 to about 6.0, or from about 6.5 to about 7.0.

In some instances, the GnRH antagonist can be teverelix ((Ac-D-NaID-pClPhe-D-Pal-Ser-Tyr-D-Hci-Leu-Lys(iPr)-Pro-D-Ala-NH₂). Teverelix is a synthetic decapeptide with a water solubility of approximately 10 mg/mL.

Formulations comprising many counter ions of teverelix can form gels. However, it is advantageous to formulate and administer a solution or microcrystalline suspension of teverelix, as such formulations can provide both immediate and prolonged release depending on the specific formulation. WO 2003/022243 discloses production of strong acid salts of teverelix, such as triflouroacetic acid and sulfuric acid. Such salts can provide release of teverelix over a prolonged period of time. Alternatively, administration of the acetate salt of teverelix can provide immediate release when administered subcutaneously. In some exemplary embodiments, the GnRH salt administered is teverelix trifluoromethanesulfonate, teverelix triflouroacetate, teverelix benesulfonate, teverelix acetate, or a combination thereof.

Teverelix TFA has inherent advantages over other GnRH antagonists, including a lower risk of local irritation or anaphylactic reactions. For example, teverelix has been shown to produce less local irritation at the administration site than degarelix, as well as display of a more dose-dependent response to administration.

In some instances, a GnRH antagonist can be peptide. In some cases, a GnRH antagonist can be in a crystalline form. In some instances, a GnRH antagonist can be in a microcrystalline form. In some instances, a GnRH antagonist can be in the form of a solution, an emulsion, a gel, or a suspension. In some exemplary embodiments, a GnRH antagonist can comprise a crystalline suspension. In some exemplary embodiments, a GnRH antagonist can comprise a microcrystalline peptide suspension.

Also disclosed herein are methods of preparing a GnRH antagonist or a salt thereof as a microcrystalline aqueous suspension comprising contacting a microcrystalline form of the GnRH antagonist with an aqueous solution or diluent.

In some cases, the GnRH antagonist can be formulated as a composition comprising an excipient, diluent, or carrier. In some cases, the composition can be a pharmaceutical composition comprising a pharmaceutically acceptable excipient, diluent, or carrier.

In some embodiments, the diluent can be water or an aqueous buffering agent. In some embodiments, the buffering agent can be saline. In some embodiments, the buffering agent can be citrate. In some embodiments, the buffering agent can be phosphate. In some embodiments, the buffering agent can be acetate. In some embodiments, the buffering agent can be glycine. In some embodiments, the buffering agent can be tris(hydroxymethyl)aminomethane (tris). In some embodiments, the buffering agent can be 4-(2-hydroxyethyl)-1-piperazineethanesulfonic acid (HEPES). In some embodiments, the buffering agent can be piperazine-N,N'-bis(2-ethanesulfonic acid) (PIPES) .

In some cases, the composition can comprise an isotonic agent. In some embodiments, the isotonic agent can be a polyhedric alcohol or a polyol. In some embodiments, the polyol does not comprise ethylene glycol. In some embodiments, the polyol can be propylene glycol. In some embodiments, the polyol can be a sugar. In some embodiments, the sugar can be sucrose. In some embodiments, the sugar can be glucose. In some embodiments, the sugar can be fructose. In some embodiments, the sugar can be maltose. In some embodiments, the polyol can be a sugar alcohol. In some embodiments, the sugar alcohol can be glycerol. In some embodiments, the sugar alcohol can be erythritol. In some embodiments, the sugar alcohol can be threitol. In some embodiments, the sugar alcohol can be arabitol. In some embodiments, the sugar alcohol can be xylitol. In some embodiments, the sugar alcohol can be ribitol. In some embodiments, the sugar alcohol can be sorbitol. In some embodiments, the sugar alcohol can be galactitol. In some embodiments, the sugar alcohol can be fucitol. In some embodiments, the sugar alcohol can be iditol. In some embodiments, the sugar alcohol can be inositol. In some embodiments, the sugar alcohol can be volemitol. In some embodiments, the sugar alcohol can be isomalt. In some embodiments, the sugar alcohol can be maltitol. In some embodiments, the sugar alcohol can be lactitol. In some embodiments, the sugar alcohol can be maltotriitol. In some embodiments, the sugar alcohol can be mannitol. In some embodiments, the sugar alcohol can be maltotetraitol. In some embodiments, the sugar alcohol can be polyglycitol.

In some exemplary embodiments, an aqueous suspension comprising a GnRH antagonist can be lyophilized. In some instances, the lyophilized formulation is stable for at least about 1 month, 2 months, 3 months, 4 months, 5 months, 6 months, 7 months, 8 months, 9 months, 10 months, 11 months, 1 year, 2 years, 3 years, 4 years, or 5 years when stored in a closed container at 10%, 20%, 30%, 40%, 50%, 60%, 70%, 80%, or 90% relative humidity at a temperature of from about 2 °C to about 30 °C, from about 2 °C to about 29 °C, from about 2 °C to about 28 °C, from about 2 °C to about 27 °C, from about 2 °C to about 26 °C, from about 2 °C to about 25 °C, from about 2 °C to about 24 °C, from about 2 °C to about 23 °C, from about 2 °C to about 22 °C, from about 2 °C to about 21 °C, from about 2 °C to about 20 °C, from about 2 °C to about 19 °C, from about 2 °C to about 18 °C, from about 2 °C to about 17 °C, from about 2 °C to about 16 °C, from about 2 °C to about 15 °C, from about 2 °C to about 14 °C, from about 2 °C to about 13 °C, from about 2 °C to about 12 °C, from about 2 °C to about 11 °C, from about 2 °C to about 10 °C, from about 2 °C to about 9 °C, from about 2 °C to about 8 °C, from about 2 °C to about 7 °C, from about 2 °C to about 6 °C, from about 2 °C to about 5 °C, from about 2 °C to about 4 °C, or from about 2 °C to about 3 °C.

A GnRH described herein can also be formulated with a microparticle. In some cases, the microparticle can be a microsphere such as a hollow microsphere used as a carrier for the therapeutic. In some instances, a GnRH antagonist can be formulated with a nanoparticle. In some instances, the microparticle or nanoparticle can be a plastic, a metal, or a polymer.

### Dosing/Administration

Disclosed herein are compositions arranged for enhancing the PK/PD profile of an administered GnRH antagonist. In some instances, a first GnRH antagonist can be administered to a subject through a first route of administration. In some instances, a second GnRH antagonist can be administered to a subject through a second route of administration. In embodiments, the administration of the first and second GnRH antagonist via the first and second route of administration, respectively, can be employed to improve the PK/PD profile of the first and second GnRH antagonist.

In some cases, the first GnRH antagonist and the second GnRH antagonist can be the same. In some cases, the first GnRH antagonist and the second GnRH antagonist can be different.

In some cases, the administration of the first GnRH antagonist and the second GnRH antagonist are performed simultaneously. In some cases, the administration of the first GnRH antagonist and the second GnRH antagonist can be separated by a period of less than 24 hours.

In some instances, the period of separation can be no more than about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 seconds. In some instances, the period of separation can be no more than about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 minutes. In some cases, the period of separation can be no more than about 1, 2, 3, 4, 5, 6, 7, 8, 9, 10, 11, 12, 13, 14, 15, 16, 17, 18, 19, 20, 21, 22, 23, or 24 hours.

In some instances, a GnRH antagonist or salt thereof described herein can be administered as a treatment course comprising a defined dosing frequency or a dosing interval. In some cases, the administration can be at a dosing frequency of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 times a month. In some instances, a GnRH antagonist or salt thereof described herein can be administered at a dosing interval of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 days apart.

In some instances, the first GnRH antagonist can be administered before the second GnRH antagonist. In some instances, the second GnRH antagonist can be administered before the first GnRH antagonist.

Administration of a GnRH antagonist described herein can be performed via any type of administration route known in the art. Examples can include injection or infusion, (including intra-arterial, intracardiac, intracerebroventricular, intradermal, intraduodenal, intramedullary, intramuscular (i.m.), intraosseous, intraperitoneal, intrathecal, intravascular, intravenous, intravitreal, epidural, and subcutaneous (s.c.)), inhalational, transdermal, transmucosal, sublingual, buccal and topical (including epicutaneous, dermal, enema, eye drops, ear drops, intranasal, and vaginal) administration. In some exemplary embodiments, the route of administration can be via an injection such as an intramuscular, intravenous, subcutaneous, or intraperitoneal injection.

In some exemplary embodiments, a GnRH antagonist or salt thereof can be administered via an intramuscular administration. In some exemplary embodiments, a GnRH antagonist can be administered via a subcutaneous administration.
In some exemplary embodiments, a first GnRH antagonist can be administered via an intramuscular administration and a second GnRH antagonist can be administered via a subcutaneous administration. Such an administration can produce a surprising and unexpected increase in useful PK parameters such as a T_{1/2} of the GnRH in circulation, which can produce a physiological result such as an increase in the duration of chemical castration brought about through administration of the GnRH antagonist.

In some cases, a GnRH antagonist or salt thereof described herein can be administered at a dose of from about 1 mg to about 480 mg, from about 5 mg to about 480 mg, from about 10 mg to about 480 mg, from about 15 mg to about 480 mg, from about 20 mg to about 480 mg, from about 25 mg to about 480 mg, from about 30 mg to about 480 mg, from about 35 mg to about 480 mg, from about 40 mg to about 480 mg, from about 45 mg to about 480 mg, from about 50 mg to about 480 mg, from about 55 mg to about 480 mg, from about 60 mg to about 480 mg, from about 65 mg to about 480 mg, from about 70 mg to about 480 mg, from about 75 mg to about 480 mg, from about 80 mg to about 480 mg, from about 85 mg to about 480 mg, from about 90 mg to about 480 mg, from about 95 mg to about 480 mg, from about 100 mg to about 480 mg, from about 105 mg to about 480 mg, from about 110 mg to about 480 mg, from about 115 mg to about 480 mg, from about 120 mg to about 480 mg, from about 125 mg to about 480 mg, from about 130 mg to about 480 mg, from about 135 mg to about 480 mg, from about 140 mg to about 480 mg, from about 145 mg to about 480 mg, from about 150 mg to about 480 mg, from about 155 mg to about 480 mg, from about 160 mg to about 480 mg, from about 165 mg to about 480 mg, from about 170 mg to about 480 mg, from about 175 mg to about 480 mg, from about 180 mg to about 480 mg, from about 185 mg to about 480 mg, from about 190 mg to about 480 mg, from about 195 mg to about 480 mg, from about 200 mg to about 480 mg, from about 205 mg to about 480 mg, from about 210 mg to about 480 mg, from about 215 mg to about 480 mg, from about 220 mg to about 480 mg, from about 225 mg to about 480 mg, from about 230 mg to about 480 mg, from about 235 mg to about 480 mg, from about 240 mg to about 480 mg, from about 245 mg to about 480 mg, from about 250 mg to about 480 mg, from about 255 mg to about 480 mg, from about 260 mg to about 480 mg, from about 265 mg to about 480 mg, from about 270 mg to about 480 mg, from about 275 mg to about 480 mg, from about 280 mg to about 480 mg, from about 285 mg to about 480 mg, from about 290 mg to about 480 mg, from about 295 mg to about 480 mg, or from about 300 mg to about 480 mg. In some cases, a GnRH antagonist or salt thereof described herein can be administered at a dose of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179 180, 181, 182, 183, 184, 184, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 242, 243, 244, 245, 246, 247, 248, 249. 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369,370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, or 480 mg.

In some cases, a GnRH antagonist or salt thereof described herein can be administered to provide a blood plasma concentration of the GnRH antagonist, metabolite, or salt thereof of from about 0.5 ng/mL to about 10 ng/mL, from about 1 ng/mL to about 10 ng/mL, from about 1.5 ng/mL to about 10 ng/mL, from about 2 ng/mL to about 10 ng/mL, from about 2.5 ng/mL to about 10 ng/mL, from about 3 ng/mL to about 10 ng/mL, from about 3.5 ng/mL to about 10 ng/mL, from about 4 ng/mL to about 10 ng/mL, from about 4.5 ng/mL to about 10 ng/mL, from about 5 ng/mL to about 10 ng/mL, from about 5.5 ng/mL to about 10 ng/mL, from about 6 ng/mL to about 10 ng/mL, from about 6.5 ng/mL to about 10 ng/mL, from about 7 ng/mL to about 10 ng/mL, from about 7.5 ng/mL to about 10 ng/mL, from about 8 ng/mL to about 10 ng/mL, from about 8.5 ng/mL to about 10 ng/mL, from about 9 ng/mL to about 10 ng/mL, or from about 9.5 ng/mL to about 10 ng/mL.

In some cases, a GnRH antagonist or salt thereof described herein can be administered to provide a blood plasma concentration of the GnRH antagonist, metabolite, or salt thereof after administration to a subject of at least about 20 ng/mL, 19.5 ng/mL, 19 ng/mL, 18.5 ng/mL, 18 ng/mL, 17.5 ng/mL, 17 ng/mL, 16.5 ng/mL, 16 ng/mL, 15.5 ng/mL, 15 ng/mL, 14.5 ng/mL, 14 ng/mL, 13.5 ng/mL, 13 ng/mL, 12.5 ng/mL, 12 ng/mL, 11.5 ng/mL, 11 ng/mL, 10.5 ng/mL, 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL.

In some cases, a GnRH antagonist or salt thereof described herein can be administered to provide a Cmax of the GnRH antagonist, metabolite, or salt thereof after administration to a subject of at least about 100 ng/mL, 95 ng/mL, 90 ng/mL, 85 ng/mL, 80 ng/mL, 75 ng/mL, 70 ng/mL, 65 ng/mL, 60 ng/mL, 55 ng/mL, 50 ng/mL, 45 ng/mL, 40 ng/mL, 35 ng/mL, 30 ng/mL, 25 ng/mL, 20 ng/mL, 15 ng/mL, 10 ng/mL, or 5 ng/mL. In some cases, a GnRH antagonist or salt thereof described herein can be administered to provide a Cmax of the GnRH antagonist, metabolite, or salt thereof of at least about 50 ng/mL, 49 ng/mL, 48 ng/mL, 47 ng/mL, 46 ng/mL, 45 ng/mL, 44 ng/mL, 43 ng/mL, 42 ng/mL, 41 ng/mL, 40 ng/mL, 39 ng/mL, 38 ng/mL, 37 ng/mL, 36 ng/mL, 35 ng/mL, 34 ng/mL, 33 ng/mL, 32 ng/mL, 31 ng/mL, 30 ng/mL, 29 ng/mL, 28 ng/mL, 27 ng/mL, 26 ng/mL, 25 ng/mL, 24 ng/mL, 23 ng/mL, 22 ng/mL, 21 ng/mL, 20 ng/mL, 19 ng/mL, 18 ng/mL, 17 ng/mL, 16 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL, 3 ng/mL, 2 ng/mL, 1 ng/mL, or 0.5 ng/mL.

In some instances, a GnRH antagonist or salt thereof described herein can be administered to provide an AUC(0-*t*) of the GnRH antagonist, metabolite, or salt thereof after administration to a subject of at least about 10,000 ng*h/mL, 9,900 ng*h/mL, 9,800 ng*h/mL, 9,700 ng*h/mL, 9,600 ng*h/mL, 9,500 ng*h/mL, 9,400 ng*h/mL, 9,300 ng*h/mL, 9,200 ng*h/mL, 9,100 ng*h/mL, 9,000 ng*h/mL, 8,900 ng*h/mL, 8,800 ng*h/mL, 8,700 ng*h/mL, 8,600 ng*h/mL, 8,500 ng*h/mL, 8,400 ng*h/mL, 8,300 ng*h/mL, 8,200 ng*h/mL, 8,100 ng*h/mL, 8,000 ng*h/mL, 7,900 ng*h/mL, 7,800 ng*h/mL, 7,700 ng*h/mL, 7,600 ng*h/mL, 7,500 ng*h/mL, 7,400 ng*h/mL, 7,300 ng*h/mL, 7,200 ng*h/mL, 7,100 ng*h/mL, 7,000 ng*h/mL, 6,900 ng*h/mL, 6,800 ng*h/mL, 6,700 ng*h/mL, 6,600 ng*h/mL, 6,500 ng*h/mL, 6,400 ng*h/mL, 6,300 ng*h/mL, 6,200 ng*h/mL, 6,100 ng*h/mL, 6,000 ng*h/mL, 5,900 ng*h/mL, 5,800 ng*h/mL, 5,700 ng*h/mL, 5,600 ng*h/mL, 5,500 ng*h/mL, 5,400 ng*h/mL, 5,300 ng*h/mL, 5,200 ng*h/mL, 5,100 ng*h/mL, 5,000 ng*h/mL, 4,900 ng*h/mL, 4,800 ng*h/mL, 4,700 ng*h/mL, 4,600 ng*h/mL, 4,500 ng*h/mL, 4,400 ng*h/mL, 4,300 ng*h/mL, 4,200 ng*h/mL, 4,100 ng*h/mL, 4,000 ng*h/mL, 3,900 ng*h/mL, 3,800 ng*h/mL, 3,700 ng*h/mL, 3,600 ng*h/mL, 3,500 ng*h/mL, 3,400 ng*h/mL, 3,300 ng*h/mL, 3,200 ng*h/mL, 3,100 ng*h/mL, 3,000 ng*h/mL, 2,900 ng*h/mL, 2,800 ng*h/mL, 2,700 ng*h/mL, 2,600 ng*h/mL, 2,500 ng*h/mL, 2,400 ng*h/mL, 2,300 ng*h/mL, 2,200 ng*h/mL, 2,100 ng*h/mL, 2,000 ng*h/mL, 1,900 ng*h/mL, 1,800 ng*h/mL, 1,700 ng*h/mL, 1,600 ng*h/mL, 1,500 ng*h/mL, 1,400 ng*h/mL, 1,300 ng*h/mL, 1,200 ng*h/mL, 1,100 ng*h/mL, 1,000 ng*h/mL, 900 ng*h/mL, 800 ng*h/mL, 700 ng*h/mL, 600 ng*h/mL, 500 ng*h/mL, 400 ng*h/mL, 300 ng*h/mL, 200 ng*h/mL, or 100 ng*h/mL, where t can be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days after administration of the GnRH antagonist.

In some exemplary embodiments, a GnRH antagonist or salt thereof described herein can be administered to provide an AUC(0-*t*) of the GnRH antagonist, metabolite, or salt thereof after administration to a subject of from about 100 ng*h/mL to about 10,000 ng*h/mL, from about 100 ng*h/mL to about 9,000 ng*h/mL, from about 100 ng*h/mL to about 8,000 ng*h/mL, from about 100 ng*h/mL to about 7,000 ng*h/mL, from about 100 ng*h/mL to about 6,000 ng*h/mL, from about 100 ng*h/mL to about 5,000 ng*h/mL, from about 100 ng*h/mL to about 4,000 ng*h/mL, from about 100 ng*h/mL to about 3,000 ng*h/mL, from about 100 ng*h/mL to about 2,000 ng*h/mL, from about 100 ng*h/mL to about 100 ng*h/mL, from about 100 ng*h/mL to about 900 ng*h/mL, from about 100 ng*h/mL to about 800 ng*h/mL, from about 100 ng*h/mL to about 700 ng*h/mL, from about 100 ng*h/mL to about 600 ng*h/mL, from about 100 ng*h/mL to about 500 ng*h/mL, from about 100 ng*h/mL to about 400 ng*h/mL, from about 100 ng*h/mL to about 300 ng*h/mL, or from about 100 ng*h/mL to about 200 ng*h/mL, where t can be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days after administration of the GnRH antagonist.

In some exemplary embodiments, a GnRH antagonist or salt thereof described herein can be administered to provide an AUC(0-*t*) of the GnRH antagonist, metabolite, or salt thereof after administration to a subject of from about 1,000 ng*h/mL to about 10,000 ng*h/mL, from about 1,000 ng*h/mL to about 9,000 ng*h/mL, from about 1,000 ng*h/mL to about 8,000 ng*h/mL, from about 1,000 ng*h/mL to about 7,000 ng*h/mL, from about 1,000 ng*h/mL to about 6,000 ng*h/mL, from about 1,000 ng*h/mL to about 5,000 ng*h/mL, from about 1,000 ng*h/mL to about 4,000 ng*h/mL, from about 1,000 ng*h/mL to about 3,000 ng*h/mL, or from about 1,000 ng*h/mL to about 2,000 ng*h/mL, where t can be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days after administration of the GnRH antagonist.

In some exemplary embodiments, a GnRH antagonist or salt thereof described herein can be administered to a subject to provide a blood plasma concentration in the subject of at least about 5 ng/mL for a time period of from about 6, 12, 18, 24, 30, 36, 42, 48, 54, 60, 66, 72, 78, 84, 90, 96, 102, 108, 114, or 120 hours after the administration.

In some instances, an additional pharmaceutical substance can be administered. In some instances, the additional pharmaceutical substance can be co-administered with the GnRH antagonist. In some instances, the additional pharmaceutical substance can be administered at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 seconds apart. In some cases, the additional pharmaceutical substance can be administered at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 minutes apart. In some cases, the additional pharmaceutical substance can be administered at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 184, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, or 240 hours apart. In some cases, the additional pharmaceutical substance can be administered at least about 1, 2, 3, 4, 5, 6, 7, 8, 9, 10, 11, 12, 13, or 14 days apart.

In some instances, the additional pharmaceutical substance can be an antineoplastic. Examples of antineoplastics can include cyclophosphamide, methotrexate, 5-fluorouracil, doxorubicin, procarbazine, prednisolone, bleomycin, vinblastine, dacarbazine, cisplatin, epirubicin, a salt of any of these, and any combination thereof.

In some instances, the additional pharmaceutical substance can be an adrenergic receptor antagonist such as an alpha blocker or a beta blocker. Examples of alpha blockers can include phenoxybenzamine, phentolamine, tolazoline, trazodone, alfuzosin, doxazosin, prazosin, tamsulosin, terazosin, silodosin, carvedilol, and labetalol. Examples of beta blockers can include propranolol, bucindolol carteolol, carvedilol, labetalol, nadolol, oxprenolol, penbutolol, pindolol, sotalol, timolol, acebutolol, atenolol, betaxolol, bisoprolol, celiprolol, esmolol, metoprolol, nebivolol, and butazamine.

In some instances, the additional pharmaceutical substance can be a 5α-reductase inhibitor. Examples of 5α-reductase inhibitors can include dutasteride, tamsulosin, finasteride, episteride, and alfatradiol. Additional examples can include herb and mineral-based inhibitors such as zinc; riboflavin (vitamin B2); azelaic acid; β-sitosterol; a polyphenol; alizarin; curcumin; a green tea catechin such as (-)-epicatechin-3-gallate and (-)-epigallo-catechin-3-gallate (EGCG); valoneic acid dilactone and gallagyldilactone isolated from the heartwood of Shorea laeviforia; Angelica koreana; Garden Balsam or Rose Balsam (Impatiens balsamina); pollen of turnip, turnip rape, fast plants, field mustard, or turnip mustard; dodder (Cuscuta reflexa); Euphorbia jolkinii; Lingzhi mushroom or Reishi mushroom (Ganoderma lucidum); ganoderic acid; Chinese Knotweed (Polygonum multiflorum); Black Pepper leaf extract (Piper nigrum); Red Stinkwood (Pygeum africanum); Saw Palmetto (Serenoa repens); a pine (Pinus sp. resin, active substance abietic acid); Ku Shen or Bitter root (Sophora flavescens); Japanese hedge parsley (Torilis japonica); Eastern Arborvitae or Northern Whitecedar (Thuja occidentalis); and Spore of Japanese climbing fern (Lygodium japonicum).

In some instances, the subject can be administered the additional pharmaceutical substance prior to the administration of the GnRH antagonist. In some instances, the subject can be administered the additional pharmaceutical substance after the administration of the GnRH antagonist.

In some exemplary embodiments, the subject can be administered testosterone prior to administration of the GnRH antagonist. In some exemplary embodiments, the subject has not been administered testosterone prior to administration of the GnRH antagonist.

In some instances, the GnRH antagonist can be administered at regular time intervals for an indefinite amount of time for the treatment of a chronic condition or disease. In some instances, the GnRH antagonist can be administered intermittently as needed.

### Indications

Disclosed herein are compositions arranged for administering a GnRH antagonist or a salt thereof to a subject. In some instances, the composition is used for treating, preventing, or at least partially ameliorating a disease or condition, or at least one symptom thereof. In some instances, the disease or condition can be benign prostatic hyperplasia; acute urinary retention; endometriosis; a cancer such as prostate, breast, or cervical cancer; a hormone imbalance; an androgen-sensitive condition; an estrogen sensitive condition; or a combination thereof. In some instances, the disease or condition may not be benign prostatic hyperplasia; acute urinary retention; endometriosis; a cancer such as prostate, breast, or cervical cancer; a hormone imbalance; an androgen-sensitive condition; an estrogen sensitive condition; or a combination thereof. Administration of a first and second GnRH antagonist via a first and second route of entry, respectively, can be used to achieve an *in vivo* biological effect. In some instances, the effect can be a suppression of a hormone. In some cases, a hormone can be testosterone or dihydrotestosterone (DHT). In some instances, a hormone can be an estrogen, an estradiol, or progesterone.

In some instances, the composition can be used to reduce a concentration of testosterone or DHT to a concentration that is less than a concentration prior to the administration. Such a composition can be used to at least partially ameliorate a disease or condition that can result from in an elevated level of testosterone or DHT. In some cases, the disease or condition can be hyperthyroidism, Grave's disease, precocious puberty, or a cancer such as prostate cancer. In some instances, the composition arranged for reducing a concentration of testosterone or DHT can be used for chemical castration. In some instances, the composition arranged for reducing a concentration of testosterone or DHT can be used as a means for birth control.

In embodiments, administration of the GnRH antagonist can produce and/or maintain a blood plasma testosterone or DHT concentration of less than about 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL. In embodiments, administration of the GnRH antagonist can produce or maintain a blood plasma testosterone or DHT concentration of about 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL. In embodiments, administration of the GnRH antagonist can produce or maintain a blood plasma testosterone or DHT concentration of from about 0.5 ng/mL to about 10 ng/mL, from about 1 ng/mL to about 10 ng/mL, from about 1.5 ng/mL to about 10 ng/mL, from about 2 ng/mL to about 10 ng/mL, from about 2.5 ng/mL to about 10 ng/mL, from about 3 ng/mL to about 10 ng/mL, from about 3.5 ng/mL to about 10 ng/mL, from about 4 ng/mL to about 10 ng/mL, from about 4.5 ng/mL to about 10 ng/mL, from about 5 ng/mL to about 10 ng/mL, from about 5.5 ng/mL to about 10 ng/mL, from about 6 ng/mL to about 10 ng/mL, from about 6.5 ng/mL to about 10 ng/mL, from about 7 ng/mL to about 10 ng/mL, from about 7.5 ng/mL to about 10 ng/mL, from about 8 ng/mL to about 10 ng/mL, from about 8.5 ng/mL to about 10 ng/mL, from about 9 ng/mL to about 10 ng/mL, or from about 9.5 ng/mL to about 10 ng/mL.

In some exemplary embodiments, administration of the GnRH antagonist can maintain a blood plasma testosterone or DHT concentration of less than 0.5 ng/mL for at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 hours after administration.

In some specific embodiments, the composition can be used to at least partially ameliorate prostate cancer. In some cases, the composition according to the invention is administrated as a part of an androgen deprivation therapy. Administration of the GnRH antagonist can decrease levels of androgens such as testosterone and DHT as described above. Because androgens can stimulate prostate cancer cells to grow, suppression of androgens can cause an amelioration of prostate cancer by at least partially slowing a rate of growth of the prostate cancer cells, or producing a reduction in a size of a prostate. In some instances, the reduction of size can be at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%. In some instances, the reduction of size can be about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%. In some instances, the reduction in size can occur after about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days. In some instances, the reduction in size can occur after about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks.

In some instances, a prostate size can be measured using an ultrasound image. In some instances, a prostate size can be measured using a magnetic resonance imaging (MRI). In some instances, a prostate size can be determined manually by means of a rectal examination. In some instances, a prostate size can be determined digitally by means of a rectal examination. In some instances, a prostate size can be determined surgically.

In some instances, administration of a GnRH antagonist can reduce a size of a prostate after administration to a subject. In some cases, the GnRH agonist can be administered along with a neoadjuvant such as an anthracycline, a taxane, 5-fluorouracil, cyclophosphamide, carboplatin, or a combination thereof. In some cases, the GnRH can be the neoadjuvant.

In some instances, administration of a GnRH antagonist can be used to treat a disease or condition resulting from an enlarged prostate other than cancer. In some instances, the disease or condition can be benign prostatic hyperplasia, acute urinary retention, or a combination thereof.

Benign prostatic hyperplasia (BPH, otherwise known as an enlarged prostate) can occur in men as they age. Symptoms of BPH can include a weak or slow urinary system, a feeling of incomplete bladder emptying, difficulty urinating, frequent urination, and/or "dribbling" of urine. A GnRH antagonist can be used to at least partially slow a rate of growth of a prostate gland, thereby ameliorating the symptoms of BPH. In some cases, the reduction in size can be a reduction in mass. In some cases, the reduction in size can be a reduction in volume. In some instances, the reduction of size can be at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%. In some instances, the reduction of size can be about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%. In some instances, the reduction in size can occur after about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days. In some instances, the reduction in size can occur after about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks.

Acute urinary retention (AUR) is a condition characterized by an inability to voluntarily pass urine. Like BPH, AUR can be a result of an increase in size of a prostate gland. In some instances, AUR can occur in conjunction with BPH or prostate cancer. A GnRH antagonist can be used to at least partially slow a rate of growth of a prostate gland, thereby ameliorating the symptoms of AUR and increasing a rate of urine flow. In some instances, the increase in urine flow can be at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%. In some instances, the increase in urine flow can be about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, or 30%. In some instances, the increase in urine flow can occur after about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days. In some instances, the increase in urine flow can occur after about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52 weeks.

In some cases, a rate or amount of urine flow can be determined using a urine flow test or assay. In some cases, the urine test can employ an electronic uroflowmeter. In some cases, the urine flow test is a urodynamic test. In some instances, the urodynamic test can comprise a measurement of urine flow rate and/or urine volume.

In some instances, treatment via administration of a GnRH antagonist described herein can decrease an amount of prostate specific antigen (PSA) in a subject. PSA is a substance made by cells in the prostate gland (both normal cells and cancer cells). PSA is mostly found in semen, but a small amount is also found in the blood. Incidence of an enlargement of a prostate, such as through a condition such as BPH or prostate cancer, can increase an amount of PSA in a subject. Therefore, PSA levels can be used as a metric for treatment of a condition resulting in the increase in PSA levels.

In embodiments, administration of the GnRH antagonist can reduce a concentration of PSA to less than about 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL. In embodiments, administration of the GnRH antagonist can reduce a concentration of PSA to about 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL. In embodiments, administration of the GnRH antagonist can reduce a concentration of PSA to an amount from about 0.5 ng/mL to about 10 ng/mL, from about 1 ng/mL to about 10 ng/mL, from about 1.5 ng/mL to about 10 ng/mL, from about 2 ng/mL to about 10 ng/mL, from about 2.5 ng/mL to about 10 ng/mL, from about 3 ng/mL to about 10 ng/mL, from about 3.5 ng/mL to about 10 ng/mL, from about 4 ng/mL to about 10 ng/mL, from about 4.5 ng/mL to about 10 ng/mL, from about 5 ng/mL to about 10 ng/mL, from about 5.5 ng/mL to about 10 ng/mL, from about 6 ng/mL to about 10 ng/mL, from about 6.5 ng/mL to about 10 ng/mL, from about 7 ng/mL to about 10 ng/mL, from about 7.5 ng/mL to about 10 ng/mL, from about 8 ng/mL to about 10 ng/mL, from about 8.5 ng/mL to about 10 ng/mL, from about 9 ng/mL to about 10 ng/mL, or from about 9.5 ng/mL to about 10 ng/mL.

In some specific embodiments, Teverelix TFA as the first and or second GnRH antagonist can provide a therapeutic benefit to achieve prolonged suppression of testosterone. Such a composition can be used as a part of an androgen deprivation therapy, which is the commonly accepted treatment of patients with hormone sensitive prostate cancer. Based on preliminary analysis of the PK data and data collected in the Phase I study using this dosing regimen, it was expected that the increased loading dose of Teverelix LA used in this study (3 injections of 90 mg 24 h apart) would increase and prolong the initial peak and consequently increase the number of subjects castrated and increase the duration of the castration period.

While the three injections of 90 mg s.c. does increase the number of subjects castrated data suggested it would be desirable to increase the release rate in the early period (loading dose) after administration of teverelix TFA so that castration would occur almost immediately, specifically within 48 to 72 hours without the flair seen with GnRH agonists and be maintained throughout the dosing interval this is especially true for the first dosing interval. While the three consecutive 90 mg doses may adequately address the therapeutic requirements it may be too cumbersome in a clinical setting. However, dual administration of teverelix through a combination of intramuscular and subcutaneous injection can have the surprising and unexpected result of providing chemical castration for a prolonged period following the initial dual administration.

In some instances, the duration of chemical castration can be at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days. In some instances, the duration of chemical castration can be at least about 1, 2, 3, 4, 5, 6, 7, 8, 9, 10, 11, or 12 weeks.

In some exemplary embodiments, the composition is used for chemical castration wherein the administration comprises:
a) an intramuscular administering of teverelix trifluoroacetate to a subject; and
b) a subcutaneous administering of teverelix trifluoroacetate to the subject,
where the first administering and the second administering are performed on the same day, and where a blood plasma testosterone concentration in the subject of less than about 0.5 ng/mL is achieved by about 48 hours after the intramuscular administration and the subcutaneous administration.

In some exemplary embodiments, the composition is used for treating benign prostatic hyperplasia, wherein the administration comprises:
a) an intramuscular administration of teverelix trifluoroacetate to a subject; and
b) a subcutaneous administration of teverelix trifluoroacetate to the subject,
where the first administration and the second administration are performed on the same day, and where an increase in urine flow in the subject of at least about 10% is achieved by about two weeks after the intramuscular administration and the subcutaneous administration.

In some instances, the composition is used for treating a disease or condition associated with an increase in blood plasma levels of an estrogen in a female subject. In some cases, administration of a GnRH antagonist can decrease the level of estrogen in the subject, thereby at least partially ameliorating the disease or condition, or a symptom thereof. In some cases, the disease or condition is a cancer such as breast cancer. In some cases, the composition can be used for reducing pain in a subject due to a condition such as endometriosis. In some instances, the estrogen can be an estradiol such as 17α-estradiol, 17β-estradiol, or ethinylestradiol.

In embodiments, administration of the GnRH antagonist can produce and/or maintain a blood plasma concentration of an estrogen of less than about 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL. In embodiments, administration of the GnRH antagonist can produce or maintain a blood plasma concentration of an estrogen of about 10 ng/mL, 9.5 ng/mL, 9 ng/mL, 8.5 ng/mL, 8 ng/mL, 7.5 ng/mL, 7 ng/mL, 6.5 ng/mL, 6 ng/mL, 5.5 ng/mL, 5 ng/mL, 4.5 ng/mL, 4 ng/mL, 3.5 ng/mL, 3 ng/mL, 2.5 ng/mL, 2 ng/mL, 1.5 ng/mL, 1 ng/mL, or 0.5 ng/mL. In embodiments, administration of the GnRH antagonist can produce or maintain a blood plasma concentration of an estrogen of from about 0.5 ng/mL to about 10 ng/mL, from about 1 ng/mL to about 10 ng/mL, from about 1.5 ng/mL to about 10 ng/mL, from about 2 ng/mL to about 10 ng/mL, from about 2.5 ng/mL to about 10 ng/mL, from about 3 ng/mL to about 10 ng/mL, from about 3.5 ng/mL to about 10 ng/mL, from about 4 ng/mL to about 10 ng/mL, from about 4.5 ng/mL to about 10 ng/mL, from about 5 ng/mL to about 10 ng/mL, from about 5.5 ng/mL to about 10 ng/mL, from about 6 ng/mL to about 10 ng/mL, from about 6.5 ng/mL to about 10 ng/mL, from about 7 ng/mL to about 10 ng/mL, from about 7.5 ng/mL to about 10 ng/mL, from about 8 ng/mL to about 10 ng/mL, from about 8.5 ng/mL to about 10 ng/mL, from about 9 ng/mL to about 10 ng/mL, or from about 9.5 ng/mL to about 10 ng/mL.

In some exemplary embodiments, administration of the GnRH antagonist can maintain a blood plasma concentration of an estrogen of less than 0.5 ng/mL for at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 hours after administration.

The novel features of embodiments are set forth with particularity in the appended claims. A better understanding of the features and advantages of the embodiments will be obtained by reference to the following detailed description that sets forth illustrative embodiments, in which the principles of the embodiments are utilized, and the accompanying drawings of which:
**FIG. 1** depicts a testosterone and teverelix time course after i.m. administration.

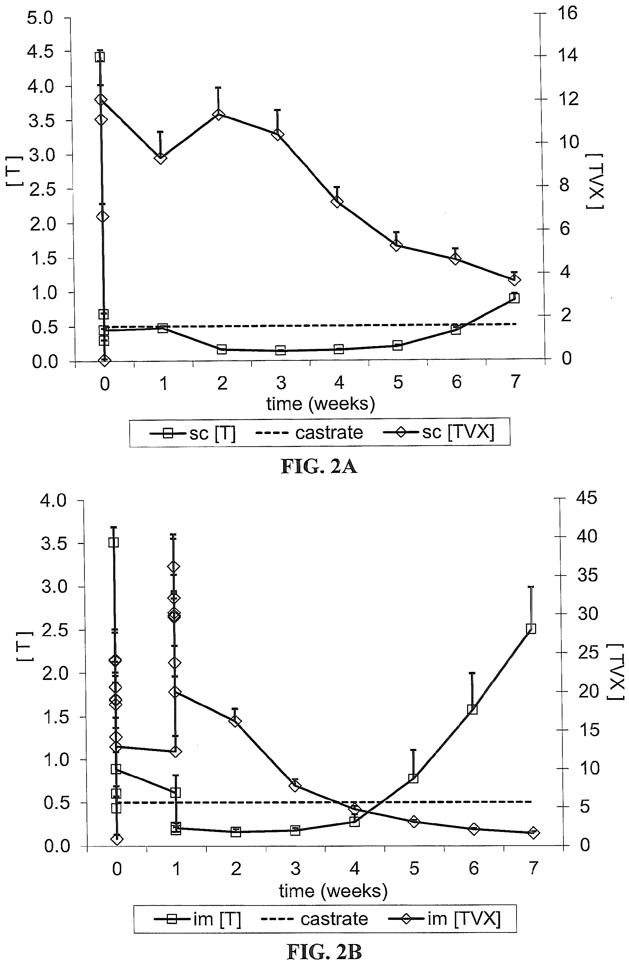

**FIG. 2A** depicts a mean concentration of teverelix over time following three s.c. 90 mg doses of teverelix.
**FIG. 2B** depicts a mean concentration of teverelix over time following two i.m. 90 mg doses of teverelix.

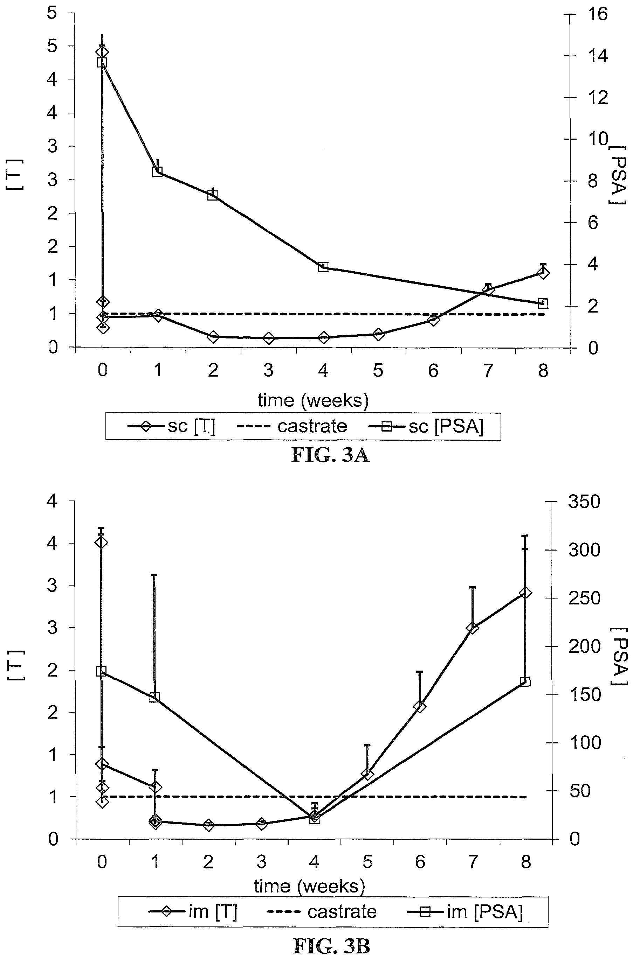

**FIG. 3A** depicts a mean concentration of PSA over time following three s.c. 90 mg doses of teverelix.
**FIG. 3B** depicts a mean concentration of PSA over time following two i.m. 90 mg doses of teverelix.
**FIG. 4** depicts an extrapolated mean concentration of teverelix over time following a single i.m. 90 mg dose of teverelix.
**FIG. 5** depicts an extrapolated mean concentration of teverelix over time following a single s.c. 90 mg dose of teverelix.
**FIG. 6** depicts a simulated PK profile of a GnRH antagonist after a 90 mg i.m. + 90 mg s.c. administration, followed by a 90 mg s.c. dosage every 56 days.
**FIG. 7** depicts a simulated PK profile of a GnRH antagonist after a 90 mg i.m. + 90 mg s.c. administration, followed by a 90 mg s.c. dosage every 42 days.
**FIG. 8** depicts a simulated PK profile of a GnRH antagonist after a 90 mg i.m. + 90 mg s.c. administration, followed by a 90 mg s.c. dosage every 28 days.
**FIG. 9** depicts a PK profile of teverelix TFA after a 90 mg i.m. + 90 mg s.c. administration, followed by a 90 mg s.c. dosage every 56 days.
**FIG. 10** depicts a PK profile teverelix TFA after a 90 mg i.m. + 90 mg s.c. administration, followed by a 90 mg s.c. dosage every 42 days.
**FIG. 11** depicts a PK profile teverelix TFA after a 90 mg i.m. + 90 mg s.c. administration, followed by a 90 mg s.c. dosage every 28 days.

### EXAMPLES

### Example 1- Teverelix s.c. Injection

In a Phase I study (D-84812-Z012), doses of up to 90 mg of Teverelix TFA (trifluoroacetate) that were administered to 32 healthy male volunteers as a single s.c. injection were tolerated well, with no critical findings regarding laboratory and clinical safety. The pharmacodynamics of teverelix showed a rapid marked suppression of LH, testosterone and, to a lesser extent, FSH. Duration of at least half-maximum testosterone suppression increased with dose. The pharmacokinetics of teverelix showed an initial increase followed by a slow and controlled release of drug. The half-life ranged between 252 h and 331 h. The maximum plasma concentrations were observed 1.75 h to 3 h after s.c. injection. Dose proportionality was demonstrated between the 60 mg and 90 mg doses of Teverelix TFA. Only the low doses of 10 mg and 30 mg Teverelix LA appeared to be insufficient to attain hormone suppression over a long period.

In a second Phase I study (EP-24332T-A004), a second injection was administered 24 h or 48 h after the first in order to increase and prolong the initial peak, as well as increase the teverelix level and consequently the number of subjects castrated. As the previous Phase I study had shown 10 mg and 30 mg to be insufficient to provide a long-term suppression, 2 doses of 60 mg or 90 mg (6 subjects per group) or placebo (4 subjects) were administered at a 24 h interval and 2 doses of 90 mg at a 48 h interval. Teverelix was tolerated well and only mild local indurations at the injection sites were observed. No critical safety findings were reported. The pharmacodynamics of teverelix showed that the mean levels of testosterone were maintained at 40% and 60% of the baseline value for more than 4 weeks in the 60 mg/60 mg and 90 mg/90 mg groups, respectively. The protocol of the study included 6 subjects who received 90 mg Teverelix TFA on D1; D2; D3. In this group, suppression of testosterone to below castration level (<0.5 ng/mL) was achieved in 5 out of 6 subjects. However, mean testosterone level was above castration level as the pharmacodynamic response in one subject was different from the pharmacodynamic response in the remaining 5 subjects.

### Example 2 - Teverelix i.m. Injection (2 x 90 mg, Days 1 and 8)

In order to test the hypothesis that and i.m. injection of a GnRH antagonist could be useful as part of a prostate cancer regimen, a Phase II Multicenter, Open-label Study Investigating the Pharmacokinetics, Pharmacodynamics, Efficacy and Safety of a Teverelix 90 mg, TFA formulation was conducted.

The primary objective was to assess the duration of action of an initial "loading" dose regimen of Teverelix TFA delivered i.m. in terms of suppression of testosterone to below castrate level (0.5 ng/mL [2 nmol/L]). The secondary objectives were to assess the pharmacodynamics of teverelix in terms of ability to suppress and to maintain plasma testosterone levels below castration level (<0.5 ng/mL [2 nmol/L]) for 3 weeks; to assess the effects on luteinising hormone (LH) and prostate specific antigen (PSA); and to assess the safety of Teverelix in terms of incidence of local and systemic tolerability (adverse events [AEs] and changes in laboratory parameters).

This was a multicenter, open-label, non-controlled study in 14 patients diagnosed with advanced prostate cancer. Patients were screened for inclusion in the study between 1 and 7 days before first administration. Each patient received 2 intramuscular (i.m.) injections of 90 mg Teverelix TFA, 7 days apart, on Study Days 1 and 8. Patients attended the hospital unit on Study Days 1, 2, 3, 5, 8, 9, 10, 12 and 14 and then weekly until, after Week 3, two consecutive, increasing determinations of testosterone concentration above castration level were recorded, with the second determination of testosterone above 2 ng/mL. Blood samples were taken for the evaluation of teverelix, testosterone and LH in plasma, and PSA levels. Safety and tolerability were assessed throughout the study by monitoring local and systemic tolerability (AEs, clinical laboratory tests, vital signs an electrocardiogram [ECG]).

### Pharmacokinetics of Teverelix

The geometric means for Cmax(obs), AUC(0-t), and AUC(0-·) (along with respective coefficients of variations presented in parenthesis); Tmax median (along with the range of Tmax values presented in parenthesis); and arithmetic mean for T_{1/2,elim} (along with standard deviation presented in parenthesis) are presented in Table 1 for each dose of teverelix i.m..

**Table 1: Plasma PK Parameter Estimates for Teverelix TFA for Day 1 and Day 8**

| Treatment | N | Cmax(obs) (ng/mL) Geometric mean (CV%) | AUC(0-t) (ng.h/mL) Geometric mean (CV%) | AUC(0-∞) (ng.h/mL) Geometric mean (CV%) | Tmax(obs) (h) Median (range) | T_{1/2,elim} (h) Arithmetic mean (SD) |
|---|---|---|---|---|---|---|
| Day 1(Dose 1): 1 x 90 mg Teverelix TFA (i.m.) | 14 | 21.5 (69.5) | 1734 (101.1) | 4610* (86.1) | 2.00 (1.00-24.00) | 166.60* (72.9) |
| Day 8 (Dose 2): 1 x 90 mg Teverelix TFA (i.m.) | 14 | 38.0 (33.8) | 8355 (22.2) | 9377 (20.0) | 1.00 (1.00-24.00) | 404.43 (113.15) |

| | | | | | | |
|---|---|---|---|---|---|---|
| Note: *Indicates n=8. | | | | | | |

The combined geometric means for AUC and AUC(∞) (along with respective coefficients of variations presented in parenthesis); and arithmetic mean for apparent clearance (CL/F) (along with standard deviation presented in parenthesis) for the two i.m. doses of teverelix are presented in Table 2.

**Table 2: Plasma PK Parameter Estimates for Teverelix**

| Treatment | N | AUC (ng.h/mL) Geometric mean (CV%) | AUC(∞) (ng.h/mL) Geometric mean (CV%) | CL/F (mL/h) Arithmetic mean (SD) |
|---|---|---|---|---|
| 2 x 90 mg Teverelix TFA (i.m.) | 14 | 10480 (27.3) | 11510 (24.6) | 14940 (3720) |

### Pharmacokinetic Conclusions

Secondary teverelix concentration maxima were apparent in concentration *vs* time profiles more than 24 h after the administration of the final dose, indicating the sustained release of drug from the long-acting teverelix formulation.

Maximum concentrations for the second dose were greater than the corresponding concentrations following the first dose (Cmax(obs)) as a result of super-imposition of quantifiable teverelix from the second dose onto the preceding concentration-time profile. This is consistent with the quantifiable predose teverelix samples observed prior to administration of the second dose.

Estimates of T_{1/2,elim} for teverelix were longer for the second dose compared with the first dose. The blood sampling period after the second dose was longer than that after the first dose as samples were taken until the criteria for the determination of the PD parameter T_{esc} were achieved. Therefore, the longer T_{1/2,elim} estimates observed for the second dose probably reflects the later time ranges used to determine the lambda-z slope for plasma profiles.

### Determination of Testosterone levels

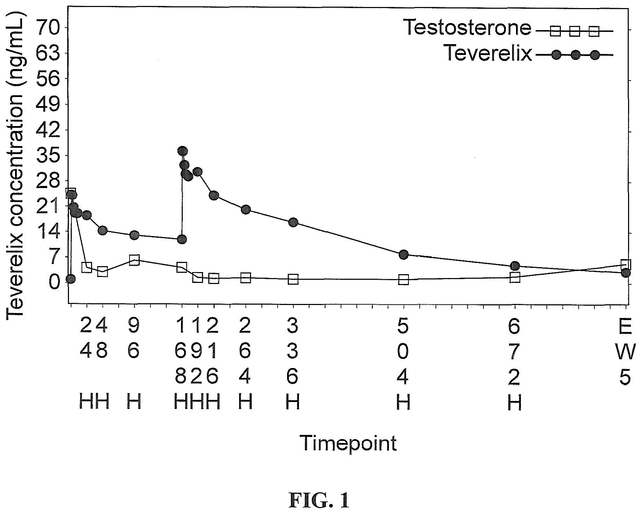

Mean concentrations of testosterone decreased rapidly after administration of teverelix (Day 2 mean [SD] change from baseline was -2.906 [0.545] ng/mL, a decrease from baseline of approximately 83%). Suppression of serum testosterone concentrations continued until at least the end of Week 5 (see Figure 1) and individual serum concentrations for all patients, except for Patient 01, remained below 2 ng/mL. Patient 01 had serum testosterone concentrations of 0.27, 1.25 and 4.97 ng/mL at the end of Weeks 3, 4 and 5, respectively.

### Determination of Teverelix (ng/mL) and Testosterone (ng/mL), Mean Values on a Linear Scale

By Day 9 (i.e. 2 days after administration of the second dose) all patients' testosterone concentration had decreased to below a threshold considered to be biochemical castration (<0.5 ng/mL); see Figure 1.

The time of onset of castration (T_{cast}), time of escape from castration (T_{esc}), and duration of castration (DC) are summarized in Table 3.

**Table 3: Pharmacodynamic Parameters T_{cast}, T_{esc} and DC: ITT and PP Datasets**

| Treatment | Statistic | T_{cast} (Days) | T_{esc} (Days) | DC (Days) |
|---|---|---|---|---|
| 2 x 90 mg Teverelix | Mean (SD) | 2.40 (2.52) | 46.17 (10.88) | 34.77 (9.28) |
| TFA (i.m.) | Median (range) | 1.30 (0.9-8.0) | 46.20 (29.4-75.2) | 34.90 (20.8-57.7) |

The mean (SD) time taken to achieve castration (T_{cast}) was 2.40 (2.52) days. T_{cast} ranged from 0.9 to 8 days and was within 2 days of administration of the first dose of teverelix for 11/14 patients. T_{cast} was longer than 2 days for 3 patients. T_{cast} for Patients 03, 06 and 09 was estimated as 8.0, 6.0 and 6.9 days, respectively.

The mean (SD) duration of castration (DC) was 34.77 (9.28) days. DC ranged from 20.8 to 57.7 days and was longer than 4 weeks for 11/14 patients. DC was estimated to be less than 4 weeks for 3 patients: DC for Patients 01, 03 and 08 was 21.3, 20.8 and 26.0 days, respectively. The mean (SD) time to escape from castration (T_{esc}) was 46.17 (10.88) days and ranged from 29.4 to 75.2 days after the first administration of Teverelix. Escape from castration followed a steady decrease in plasma teverelix concentration.

Eight out of the 14 patients (57.1%) experienced transient breakthrough episodes during castration where concentrations of testosterone increased to above 0.5 ng/mL (Patients 02, 03, 05, 06, 07, 12, 13 and 14). In all instances, testosterone concentrations remained below 2 ng/mL and did not proceed to meet the criteria for escape from castration. For 6/8 patients, breakthrough episodes occurred before administration of the second dose of Teverelix LA, and all episodes occurred before the end of Week 2. Breakthrough episodes varied in duration from between 2.3 and 5.4 days (mean [SD] duration = 3.60 [1.28] days).

Comparison of s.c. injection 90 mg days 1, 2 and 3 vs. i.m. injection day 1 and 8.

**Table 4: Summary of Clinical Studies**

| ***Summary*** | | |
|---|---|---|
| **Param eter** | **A013** | **A014** |
| **Onset of castration** | 1.77 days | 2.40 days |
| **Mean duration of castration** | 55.32 days (range 4 - 14 weeks) | 34.77 days (range 3 - 8 weeks) |
| **Mean escape from castration** | 70.14 days | 46.17 days |
| **T below baseline** | ≥ End Week 6 | ≥ End Week 4 |
| **LH** | Decreased following first administration of teverelix on Day 1 and remained below baseline until at least the end of Week 6 | Decreased appreciably following administration of Teverelix LA and remained below baseline values until at least the end of Week 5 (LH) and Week 4 (PSA) |
| **PSA** | | |
| **Tolerability** | Very good/good for all patients | |

From the above it is clear that both the 3 x 90 mg s.c. dosing regimen and the 2 x 90mg i.m. dosing regimen would meet the primary objective of castrating the prostate cancer patients within 48 to 72 hours and maintain castration for the full 4 week dosing interval. However, it would not meet the practical criterion of being able to treat the patient with a single visit once every 4 weeks.

### Example 3 - GnRH i.m./s.c. Combination (2 x 90 mg, Day 1)

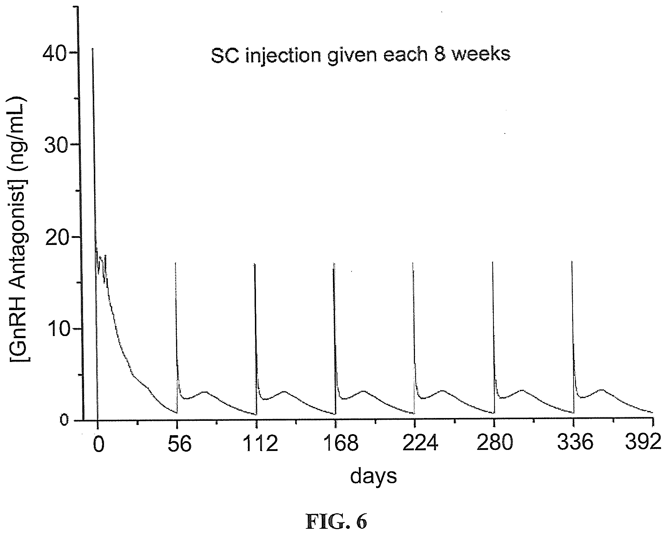

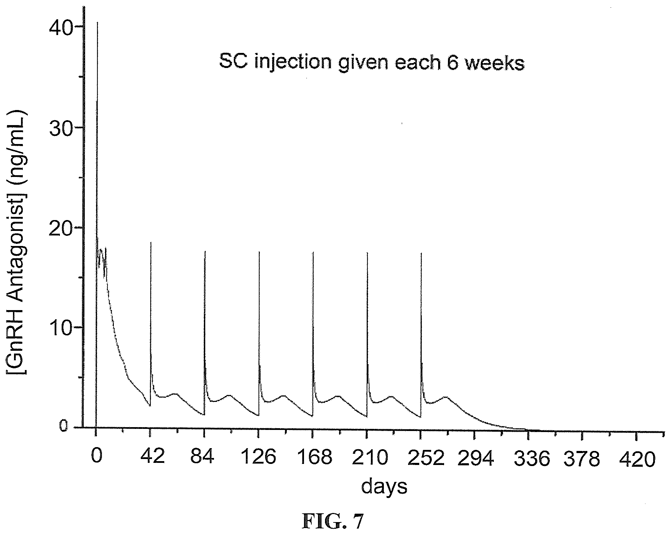

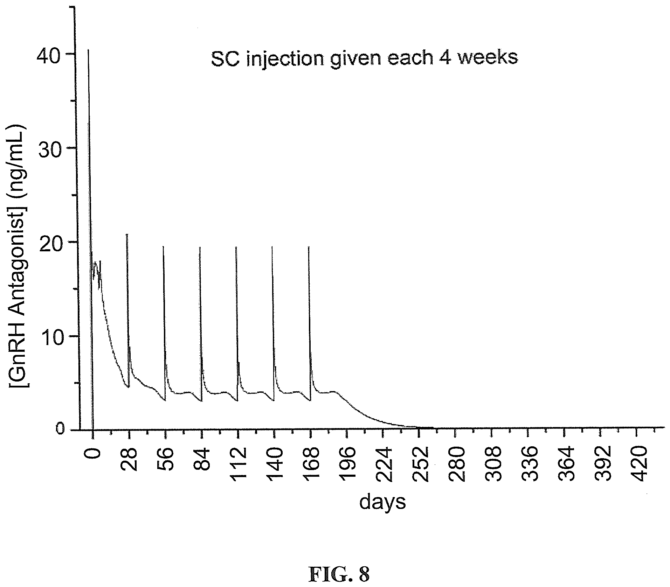

Mean data from the i.m. and s.c. injection studies was used to predict the PK profile for a combined i.m. and s.c. dosing regimen of an administration of a GnRH antagonist. A PK profile for three potential dosing regimens was constructed: Scenario A (90 mg i.m. + 90 mg s.c., then 90 mg s.c. every 56 days; Figure 6); Scenario B (90 mg i.m. + 90 mg s.c., then 90 mg s.c. every 42 days; Figure 7); and Scenario C (90 mg i.m. + 90 mg s.c., then 90 mg s.c. every 28 days; Figure 8). The steady state Cmax, Cmin, AUC and Cavg are summarized below:

**Table 5: Simulated PK/PD parameters for s.c. and i.m. dual injection**

| | **C_{max,ss} (ng/mL)** | **C_{min,ss} (ng/mL)** | **AUCₛₛ (ng × days/mL)** | **C_{avg,ss} (ng/mL)** |
|---|---|---|---|---|
| **Scenario A** | 16.95 | 0.56 | 115.57 | 2.06 |
| **Scenario B** | 17.66 | 1.27 | 115.57 | 2.75 |
| **Scenario C** | 19.33 | 2.95 | 115.57 | 4.13 |

The combined administration of the i.m. and s.c. injection, serves to provide efficient serum concentration already during the first dosing interval. As expected, shortening of the steady state dosing interval can linearly increase the average serum concentrations.

In a preferred embodiment it is preferred that the dual administration of the composition results in a PK profile substantially as depicted in Figure 8 after a first administration comprising an administering intramuscularly and an administering subcutaneously of the GnRH antagonist or salt thereof to the subject; followed by a second administration comprising an administering subcutaneously of the GnRH antagonist or salt thereof to the subject; where the second administration can be performed about 28 days after the first administration.

### Example 4 - Teverelix i.m./s.c. Combination (2 x 90 mg, Day 1)

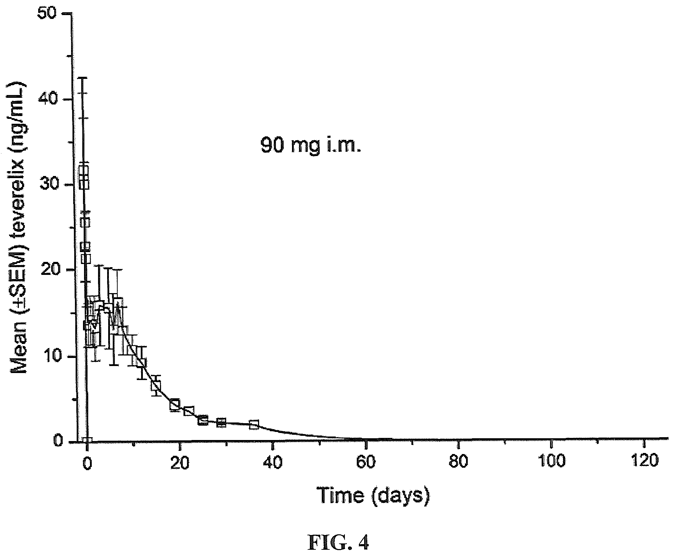

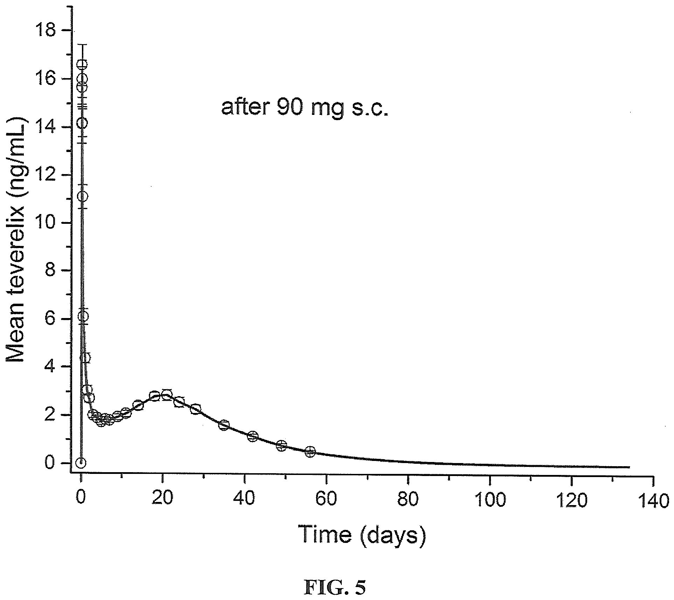

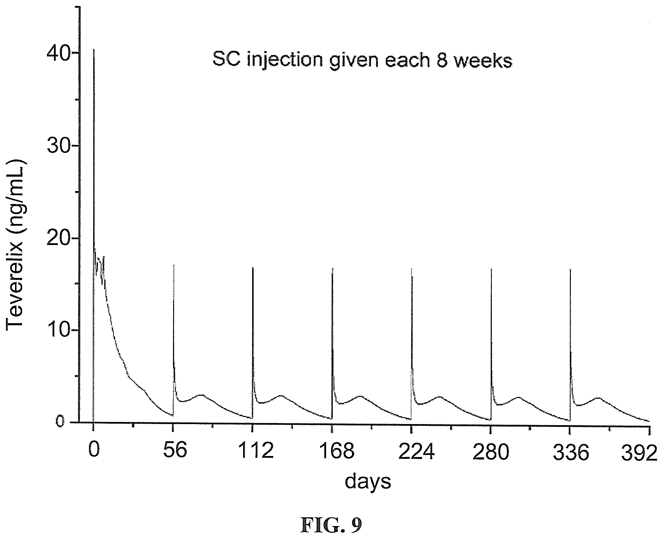

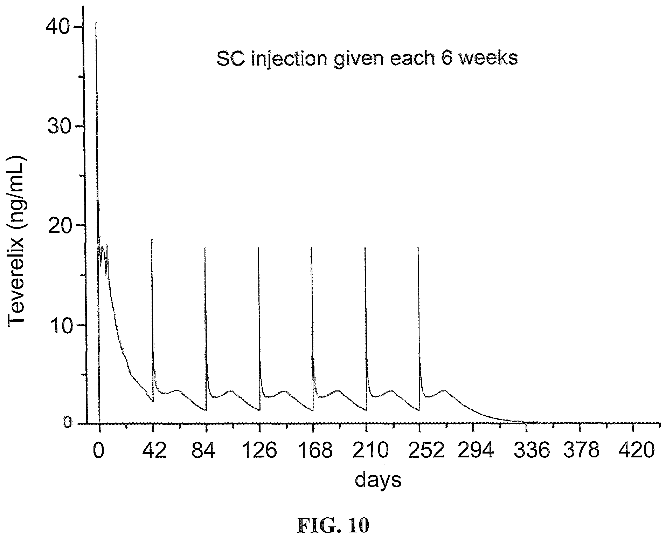

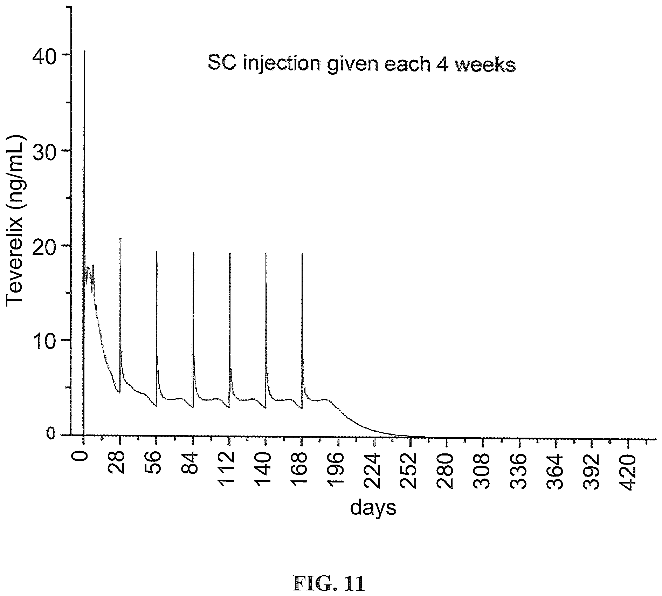

Mean data from the i.m. and s.c. injection studies was used to predict the PK profile for a combined i.m. and s.c. dosing regimen. Figures 4 and 5 depict the mean serum concentrations of teverlix (ng/mL) after a single i.m. and s.c. injection, respectively. This data was used to construct a PK profile for three potential dosing regimens: Scenario A (90 mg i.m. + 90 mg s.c., then 90 mg s.c. every 56 days; Figure 9); Scenario B (90 mg i.m. + 90 mg s.c., then 90 mg s.c. every 42 days; Figure 10); and Scenario C (90 mg i.m. + 90 mg s.c., then 90 mg s.c. every 28 days; Figure 11). The steady state Cmax, Cmin, AUC and Cavg are summarized below:

**Table 5: Simulated PK/PD parameters for s.c. and i.m. dual injection of Teverelix TFA**

| | **Cmax, ss (ng/mL)** | **Cmin, ss (ng/mL)** | **AUCss (ng** × | **Cavg, ss (ng/mL)** |
|---|---|---|---|---|
| **Scenario** | 16.95 | 0.56 | 115.57 | 2.06 |
| **Scenario** | 17.66 | 1.27 | 115.57 | 2.75 |
| **Scenario** | 19.33 | 2.95 | 115.57 | 4.13 |

The combined administration of the i.m. and s.c. injection of teverelix TFA serves to provide efficient serum concentration already during the first dosing interval. As expected, shortening of the steady state dosing interval can linearly increase the average serum concentrations

While exemplary embodiments have been shown and described herein, it will be obvious to those skilled in the art that such embodiments are provided by way of example only. Numerous variations, changes, and substitutions will occur to those skilled in the art. It should be understood that various alternatives to the embodiments described herein may be employed in as far as covered by the scope of the claims. It is intended that the following claims define the scope of the invention and that compositions for use within the scope of these claims be covered thereby.

## Claims

1. A composition comprising at least one GnRH antagonist or a salt thereof for use in the treatment of a condition or disease related to the release of a gonadotropin hormone, wherein the administration of said composition comprises independently administering a therapeutically effective amount of a first GnRH antagonist or a salt thereof via a first route of administration, and a therapeutically effective amount of a second GnRH antagonist or a salt thereof via a second route of administration, and wherein the first and the second route of administration are different, and wherein the first and second GnRH antagonist or a salt thereof, are administered substantially simultaneously or within a period of not more than 24 hours, and wherein the first route of administration is subcutaneously and the second route of administration is intramuscularly.

2. The composition for use according to claim 1, wherein the therapeutically effective amount of the first GnRH antagonist or a salt, and the therapeutically effective amount of the second GnRH antagonist or a salt thereof each independently comprise a dose ranging from about 30 mg to about 240 mg of the respective GnRH antagonist(s) or salt thereof, preferably a dosage of about 90 mg of the respective GnRH antagonist(s) or salt thereof.

3. The composition for use according to claim 1 or 2, wherein the first and the second GnRH antagonist or salt thereof are the same.

4. The composition for use according to claim 1 or 2, wherein the first and the second GnRH antagonist are different.

5. The composition for use according to any of the preceding claims, wherein the administration of the first and second GnRH antagonist or salt thereof produces a maximal plasma concentration (Cmax) of at least about 10 ng/mL and an area under the plasma concentration versus time curve from time 0 to time t (AUC₍₀₋ₜ₎) from about 100 ng*h/mL to about 8,000 ng*h/mL when administered at a total dose of from about 60 mg to about 480 mg, and wherein t is at least about 28 days.

6. The composition for use according to any of the preceding claims, wherein the administration comprising administering an additional dosage of a therapeutically effective amount of an GnRH antagonist or salt thereof at a dosing interval of at least about 28 days.

7. The composition for use according to claim 6, wherein the therapeutically effective amount of the additional dosage of the GnRH antagonist or salt additional dosage is of about 90 mg of the GnRH antagonist or salt thereof.

8. The composition for use according to any of the preceding claims, wherein the GnRH antagonist or salt thereof is a peptide or salt thereof and preferably comprises a crystalline form, wherein the crystalline form is in suspension in a liquid, preferably a microcrystalline suspension in a liquid.

9. The composition for use according to any of the preceding claims, wherein the GnRH antagonist or salt is selected from the group comprising abarelix, cetrorelix, degarelix, ganirelix, ozarelix, antide, teverelix, or a salt of any of the above.

10. The composition for use according to any of the preceding claims, wherein the GnRH antagonist or salt thereof is a teverelix salt, preferably teverelix trifluoroacetate.

11. The composition for use according to any of the preceding claims, wherein a blood plasma teverelix concentration in the subject after administration of a therapeutically effective amount of the composition of at least about 9 ng/mL is provided.

12. The composition for use according to any of the preceding claims, wherein a maximal plasma concentration (Cmax) of teverelix in the subject of at least about 19 ng/mL is obtained after administration of a therapeutically effective amount of the composition.

13. The composition for use according to any of the preceding claims, wherein said composition is arranged for maintaining a blood plasma teverelix concentration in the subject above at least about 5 ng/mL from about: 24, 48, or 72 hours after the administering intramuscularly and the administering subcutaneously until an additional administering of an GnRH antagonist or a salt thereof.

14. The composition for use according to any of the preceding claims, wherein the condition or disease is selected from benign prostatic hyperplasia; acute urinary retention; endometriosis; a cancer such as prostate, breast, or cervical cancer; a hormone imbalance; an androgen-sensitive condition; an estrogen sensitive condition; or a combination thereof.

15. The composition for use according to any of the preceding claims, wherein the administering of the composition provide a blood plasma testosterone concentration in a male subject less than about: 3 ng/mL, 2.5 ng/mL, 2 ng/mL, 1.5 ng/mL, 1 ng/mL, or 0.5 ng/mL as measured by a liquid chromatography/mass spectrometry (LC-MS) assay.

16. The composition for use according to any of the preceding claims, wherein the blood plasma estrogen level in a female subject is reduced after the administration of the composition, relative to a blood plasma estrogen level before said administration.

17. A pharmaceutical formulation comprising the composition according to any of the claims 1 - 16 for use in the treatment according to any of the claims 1 - 16.

18. The pharmaceutical formulation for use according to claim 17, wherein said formulation further comprises at least one additional pharmaceutical substance, preferably selected from the group comprising antineoplastics, adrenergic receptor antagonists and 5α-reductase inhibitors.

19. A kit comprising the composition according to any of the claims 1 - 16 or a pharmaceutical formulation according to any of the claims 17 or 18 for use according to any of the claims 1 - 18, wherein said kit further comprise means to perform a first route of administration, and a second route of administration, as well as instructions for performing said administrations.

20. The kit for use according to claim 19, wherein the means for performing a a first route of administration, and a second route of administration comprises at least one syringe and needle for administration of the first and second GnRH antagonist.

## Patentansprüche

1. Zusammensetzung, die mindestens einen GnRH-Antagonisten oder ein Salz davon umfasst, zur Verwendung bei der Behandlung eines Zustands oder einer Krankheit, die mit der Freisetzung eines Gonadotropin-Hormons zusammenhängt, wobei die Verabreichung der Zusammensetzung die unabhängige Verabreichung einer therapeutisch wirksamen Menge eines ersten GnRH-Antagonisten oder eines Salzes davon über einen ersten Verabreichungsweg und einer therapeutisch wirksamen Menge eines zweiten GnRH-Antagonisten oder eines Salzes davon über einen zweiten Verabreichungsweg umfasst, und wobei der erste und der zweite Verabreichungsweg verschieden sind, und wobei der erste und zweite GnRH-Antagonist oder ein Salz davon im Wesentlichen gleichzeitig oder innerhalb eines Zeitraums von nicht mehr als 24 Stunden verabreicht wird, und wobei der erste Verabreichungsweg subkutan und der zweite Verabreichungsweg intramuskulär ist.

2. Zusammensetzung zur Verwendung nach Anspruch 1, wobei die therapeutisch wirksame Menge des ersten GnRH-Antagonisten oder eines Salzes und die therapeutisch wirksame Menge des zweiten GnRH-Antagonisten oder eines Salzes davon jeweils unabhängig voneinander eine Dosis im Bereich von etwa 30 mg bis etwa 240 mg des/der jeweiligen GnRH-Antagonisten oder eines Salzes davon, vorzugsweise eine Dosis von etwa 90 mg des/der jeweiligen GnRH-Antagonisten oder eines Salzes davon umfassen.

3. Zusammensetzung zur Verwendung nach Anspruch 1 oder 2, wobei der erste und der zweite GnRH-Antagonist oder ein Salz davon gleich sind.

4. Zusammensetzung zur Verwendung nach Anspruch 1 oder 2, wobei der erste und der zweite GnRH-Antagonist verschieden sind.

5. Zusammensetzung zur Verwendung nach einem der vorhergehenden Ansprüche, wobei die Verabreichung des ersten und zweiten GnRH-Antagonisten oder eines Salzes davon eine maximale Plasmakonzentration (Cmax) von mindestens etwa 10 ng/ml und eine Fläche unter der Kurve der Plasmakonzentration über der Zeit von Zeit 0 bis Zeit *t* (AUC₍₀₋ₜ₎) von etwa 100 ng*h/ml bis etwa 8.000 ng*h/ml erzeugt, wenn sie mit einer Gesamtdosis von etwa 60 mg bis etwa 480 mg verabreicht wird, und wobei *t* mindestens etwa 28 Tage beträgt.

6. Zusammensetzung zur Verwendung nach einem der vorhergehenden Ansprüche, wobei die Verabreichung die Verabreichung einer zusätzlichen Dosierung einer therapeutisch wirksamen Menge eines GnRH-Antagonisten oder eines Salzes davon in einem Dosierungsintervall von mindestens etwa 28 Tagen umfasst.

7. Zusammensetzung zur Verwendung nach Anspruch 6, wobei die therapeutisch wirksame Menge der zusätzlichen Dosierung des GnRH-Antagonisten oder der zusätzlichen Dosierung des Salzes etwa 90 mg des GnRH-Antagonisten oder des Salzes davon beträgt.

8. Zusammensetzung zur Verwendung nach einem der vorhergehenden Ansprüche, wobei der GnRH-Antagonist oder ein Salz davon ein Peptid oder ein Salz davon ist und vorzugsweise eine kristalline Form umfasst, wobei die kristalline Form in Suspension in einer Flüssigkeit, vorzugsweise einer mikrokristallinen Suspension in einer Flüssigkeit, vorliegt.

9. Zusammensetzung zur Verwendung nach einem der vorhergehenden Ansprüche, wobei der GnRH-Antagonist oder das Salz ausgewählt ist aus der Gruppe, umfassend Abarelix, Cetrorelix, Degarelix, Ganirelix, Ozarelix, Antidelix, Teverelix oder ein Salz einer der oben genannten.

10. Zusammensetzung zur Verwendung nach einem der vorhergehenden Ansprüche, wobei der GnRH-Antagonist oder ein Salz davon ein Teverelix-Salz, vorzugsweise Teverelix-Trifluoracetat, ist.

11. Zusammensetzung zur Verwendung nach einem der vorhergehenden Ansprüche, wobei eine Blutplasma-Teverelix-Konzentration in dem Subjekt nach Verabreichung einer therapeutisch wirksamen Menge der Zusammensetzung von mindestens etwa 9 ng/ml bereitgestellt wird.

12. Zusammensetzung zur Verwendung nach einem der vorhergehenden Ansprüche, wobei eine maximale Plasmakonzentration (Cmax) von Teverelix in dem Subjekt von mindestens etwa 19 ng/ml nach Verabreichung einer therapeutisch wirksamen Menge der Zusammensetzung erhalten wird.

13. Zusammensetzung zur Verwendung nach einem der vorhergehenden Ansprüche, wobei die Zusammensetzung zur Aufrechterhaltung einer Blutplasma-Teverelix-Konzentration in dem Subjekt oberhalb von mindestens etwa 5 ng/ml von etwa: 24, 48 oder 72 Stunden nach der intramuskulären Verabreichung und der subkutanen Verabreichung bis zu einer zusätzlichen Verabreichung eines GnRH-Antagonisten oder eines Salzes davon ausgelegt ist.

14. Zusammensetzung zur Verwendung nach einem der vorhergehenden Ansprüche, wobei der Zustand oder die Krankheit ausgewählt ist aus benigner Prostatahyperplasie; akutem Harnverhalt; Endometriose; einem Krebs wie Prostata-, Brust- oder Gebärmutterhalskrebs; einem Hormonungleichgewicht; einem androgensensitiven Zustand; einem östrogensensitivem Zustand; oder einer Kombination davon.

15. Zusammensetzung zur Verwendung nach einem der vorhergehenden Ansprüche, wobei die Verabreichung der Zusammensetzung bei einem männlichen Probanden eine Blutplasma-Testosteron-Konzentration von weniger als etwa: 3 ng/ml, 2,5 ng/ml, 2 ng/ml, 1,5 ng/ml, 1 ng/ml oder 0,5 ng/ml bereitstellt, gemessen mit einem Flüssigchromatographie/Massenspektrometrie(LC-MS)-Assay.

16. Zusammensetzung zur Verwendung nach einem der vorhergehenden Ansprüche, wobei der Blutplasma-Östrogenspiegel bei einem weiblichen Subjekt nach der Verabreichung der Zusammensetzung im Vergleich zu einem Blutplasma-Östrogenspiegel vor der Verabreichung reduziert ist.

17. Pharmazeutische Formulierung, umfassend die Zusammensetzung nach einem der Ansprüche 1 - 16 zur Verwendung bei der Behandlung nach einem der Ansprüche 1 - 16.

18. Pharmazeutische Formulierung zur Verwendung nach Anspruch 17, wobei die Formulierung ferner mindestens eine zusätzliche pharmazeutische Substanz enthält, vorzugsweise ausgewählt aus der Gruppe, umfassend Antineoplastika, adrenerge Rezeptorantagonisten und 5α-Reduktase-Inhibitoren.

19. Kit, umfassend die Zusammensetzung nach einem der Ansprüche 1 - 16 oder eine pharmazeutische Formulierung nach einem der Ansprüche 17 oder 18 zur Verwendung nach einem der Ansprüche 1 - 18, wobei das Kit ferner Mittel zur Ausführung eines ersten Verabreichungsweges und eines zweiten Verabreichungsweges sowie Anweisungen zur Ausführung der Verabreichungen umfasst.

20. Kit zur Verwendung nach Anspruch 19, wobei die Mittel zur Ausführung eines ersten Verabreichungsweges und eines zweiten Verabreichungsweges mindestens eine Spritze und eine Nadel zur Verabreichung des ersten und zweiten GnRH-Antagonisten umfassen.

## Revendications

1. Composition qui comprend au moins un antagoniste de la GnRH ou un de ses sels pour son utilisation dans le traitement d'un état ou d'une maladie apparenté à la libération d'une hormone de gonadotrophine ; dans laquelle l'administration de ladite composition comprend le fait d'administrer de manière indépendante une quantité thérapeutiquement efficace d'un premier antagoniste de la GnRH ou d'un de ses sels par l'intermédiaire d'une première voie d'administration, et une quantité thérapeutiquement efficace d'un deuxième antagoniste de la GnRH ou d'un de ses sels par l'intermédiaire d'une deuxième voie d'administration ; et dans laquelle la première et la deuxième voie d'administration sont différentes ; et dans laquelle le premier et le deuxième antagoniste de la GnRH ou un de leurs sels, sont administrés de manière essentiellement simultanée ou bien au cours d'un laps de temps qui n'est pas supérieur à 24 heures ; et dans laquelle la première voie d'administration représente une voie sous-cutanée et la deuxième voie d'administration représente une voie intramusculaire.

2. Composition pour son utilisation selon la revendication 1, dans laquelle la quantité thérapeutiquement efficace du premier antagoniste de la GnRH ou d'un de ses sels, et la quantité thérapeutiquement efficace du deuxième antagoniste de la GnRH ou d'un de ses sels comprennent, chacune de manière indépendante, une dose qui se situe dans une plage allant d'environ 30 mg à environ 240 mg de l'antagoniste respectif de la GnRH ou d'un de leurs sels ; de préférence, une posologie d'environ 90 mg de l'antagoniste respectif de la GnRH ou d'un de ses sels.

3. Composition pour son utilisation selon la revendication 1 ou 2, dans laquelle le premier et le deuxième antagoniste de la GnRH ou leurs sels sont identiques.

4. Composition pour son utilisation selon la revendication 1 ou 2, dans laquelle le premier et le deuxième antagoniste de la GnRH sont différents.

5. Composition pour son utilisation selon l'une quelconque des revendications précédentes, dans laquelle l'administration du premier et du deuxième antagoniste de la GnRH ou de leurs sels permet d'obtenir une concentration plasmatique maximale (Cmax) qui s'élève à au moins environ 10 ng/ml et une aire sous la courbe de la concentration plasmatique par rapport au temps à partir du temps 0 jusqu'au temps *†* (AUC_{(0-†)}) qui s'élève d'environ 100 ng*h/ml à environ 8000 ng*h/ml, lorsque l'administration s'effectue à une dose totale qui s'élève d'environ 60 mg à environ 480 mg, et dans laquelle t représente au moins environ 28 jours.

6. Composition pour son utilisation selon l'une quelconque des revendications précédentes, dans laquelle l'administration comprend le fait d'administrer une posologie supplémentaire d'une quantité thérapeutiquement efficace d'un antagoniste de la GnRH ou d'un de ses sels à un intervalle posologique d'au moins environ 28 jours.

7. Composition pour son utilisation selon la revendication 6, dans laquelle la quantité thérapeutiquement efficace de la posologie supplémentaire de l'antagoniste de la GnRH ou de la posologie supplémentaire d'un de ses sels représente environ 90 mg de l'antagoniste de la GnRH ou d'un de ses sels.

8. Composition pour son utilisation selon l'une quelconque des revendications précédentes, dans laquelle l'antagoniste de la GnRH ou un de ses sels représente un peptide ou un de ses sels et comprend de préférence une forme cristalline ; dans laquelle la forme cristalline représente une suspension dans un liquide ; de préférence, une suspension microcristalline dans un liquide.

9. Composition pour son utilisation selon l'une quelconque des revendications précédentes, dans laquelle l'antagoniste de la GnRH ou son sel est choisi parmi le groupe qui comprend : l'abarelix, le cétrorélix, le dégarélix, le ganirélix, l'ozarélix, l'antide, le tévérélix, ou un sel de l'un quelconque des antagonistes cités ci-dessus.

10. Composition pour son utilisation selon l'une quelconque des revendications précédentes, dans laquelle l'antagoniste de la GnRH ou son sel représente un sel de tévérélix ; de préférence, le trifluoroacétate de tévérélix.

11. Composition pour son utilisation selon l'une quelconque des revendications précédentes, dans laquelle on obtient une concentration de tévérélix dans le plasma sanguin du sujet qui a fait l'objet d'une administration d'une quantité thérapeutiquement efficace de la composition, qui s'élève au moins environ 9 ng/ml.

12. Composition pour son utilisation selon l'une quelconque des revendications précédentes, dans laquelle on obtient une concentration plasmatique maximale (Cmax) de tévérélix dans le sujet, qui s'élève à au moins environ 19 ng/ml, après l'administration d'une quantité thérapeutiquement efficace de la composition.

13. Composition pour son utilisation selon l'une quelconque des revendications précédentes, dans laquelle ladite composition est conçue pour le maintien d'une concentration de tévérélix dans le plasma sanguin du sujet qui est supérieure à au moins environ 5 ng/ml à partir d'environ : 24, 48 ou 72 heures après l'administration par voie intramusculaire et après l'administration par voie sous-cutanée jusqu'à une administration supplémentaire d'un antagoniste de la GnRH ou d'un de ses sels.

14. Composition pour son utilisation selon l'une quelconque des revendications précédentes, dans laquelle l'affection ou la maladie est choisie parmi : une hyperplasie bénigne de la prostate ; une rétention urinaire aiguë ; une endométriose ; un cancer tel qu'un cancer de la prostate, du sein ou du col de l'utérus ; un déséquilibre hormonal ; une affection sensible aux androgènes ; une affection sensible aux œstrogènes ; ou une de leurs combinaisons.

15. Composition pour son utilisation selon l'une quelconque des revendications précédentes, dans laquelle l'administration de la composition permet d'obtenir une concentration de testostérone dans le plasma sanguin chez un sujet de sexe masculin, qui est inférieure à environ : 3 ng/ml, 2,5 ng/ml, 2 ng/ml, 1,5 ng/ml, 1 ng/ml ou 0,5 ng/ml, telle qu'on la mesure par l'intermédiaire d'une analyse qui fait appel à un couplage d'une chromatographie en phase liquide et d'une spectrométrie de masse (LC-MS).

16. Composition pour son utilisation selon l'une quelconque des revendications précédentes, dans laquelle le taux d'oestrogènes dans le plasma sanguin chez un sujet de sexe féminin est réduit après l'administration de la composition, par rapport à un taux d'oestrogènes dans le plasma sanguin qui prévaut avant ladite administration.

17. Formulation pharmaceutique qui comprend la composition selon l'une quelconque des revendications 1 à 16 pour son utilisation dans le traitement en conformité avec l'une quelconque des revendications 1 à 16.

18. Formulation pharmaceutique pour son utilisation selon la revendication 17, dans laquelle ladite formulation comprend en outre au moins une substance pharmaceutique supplémentaire, qui est choisie de manière préférentielle parmi le groupe qui comprend : des substances antinéoplasiques, des antagonistes des récepteurs adrénergiques et des inhibiteurs de la 5a-réductase.

19. Nécessaire qui comprend la composition selon l'une quelconque des revendications 1 à 16 ou formulation pharmaceutique selon l'une quelconque des revendications 17 ou 18, pour son utilisation en conformité avec l'une quelconque des revendications 1 à 18, dans lequel ledit nécessaire comprend en outre des moyens qui permettent d'envisager une première voie d'administration et une deuxième voie d'administration, de même qu'une notice d'informations de l'utilisateur pour la mise en œuvre desdites administrations.

20. Nécessaire pour son utilisation selon la revendication 19, dans lequel les moyens qui sont utilisés pour la mise en œuvre d'une première voie d'administration et d'une deuxième voie d'administration comprennent au moins une seringue et une aiguille pour l'administration du premier et du deuxième antagoniste de la GnRH.
